(19) Europäisches Patentamt / European Patent Office / Office européen des brevets

(11) **EP 4 169 607 B1**

(12) **EUROPÄISCHE PATENTSCHRIFT**

(45) Veröffentlichungstag und Bekanntmachung des Hinweises auf die Patenterteilung:
**21.01.2026 Patentblatt 2026/04**

(21) Anmeldenummer: **21204086.9**

(22) Anmeldetag: **21.10.2021**

(51) Internationale Patentklassifikation (IPC):
**B01F 23/10** *(2022.01)* **G01N 33/22** *(2006.01)*

(52) Gemeinsame Patentklassifikation (CPC):
**G01N 33/225**

(54) **MISCHANTEILSBESTIMMUNG BEIM MISCHEN VON GASEN**

DETERMINATION OF THE PROPORTIONS WHEN MIXING GASES

DÉTERMINATION DE LA PROPORTION DE MÉLANGE LORS DU MÉLANGE DES GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**26.04.2023 Patentblatt 2023/17**

(73) Patentinhaber: **MEMS AG**
**5200 BRUGG (CH)**

(72) Erfinder:
• **Prêtre, Philippe**
**5405 Dättwil (CH)**
• **Kempe, Andreas**
**8049 Zürich 8049 (CH)**

(74) Vertreter: **IPS Irsch AG**
**Langfeldstrasse 88**
**8500 Frauenfeld (CH)**

(56) Entgegenhaltungen:
EP-A1- 1 947 450     EP-A1- 2 667 276
EP-A1- 2 806 271     EP-A1- 3 098 592
EP-A1- 3 683 500

**Beschreibung**

Die Erfindung bezieht sich auf ein Verfahren und eine Messvorrichtung zur Messung des Mischverhältnisses von Gasen auch bei zeitlich ändernder Zusammensetzung der zu mischenden Gase, ohne dabei die Kalibrierung der zur Bestimmung des Mischverhältnisses verwendeten Sensoren auf die jeweilige Änderung anpassen zu müssen. Die Erfindung bezieht sich weiter auf eine Mischvorrichtung, in der das Verfahren und/oder die Messvorrichtung eingesetzt werden.

Das Mischen von Gasen kommt in der Technik an vielen Stellen vor, sei es in der Prozessindustrie zur Herstellung von kundenspezifischen Gasmischungen, in der Medizin zum Einstellen von unterschiedlichen Anästhesiegraden des Beatmungsgases oder in der Erdgasindustrie zur Bereitstellung einer gleichbleibenden Gasqualität.

Gleichbleibende Erdgasqualität wird heute z.B. dadurch gewährleistet, dass je nach Herkunft des Erdgases dieses mit Luft verdünnt auf einen tieferen oder mit Propan/Butan angereichert auf einen höheren Brennwert eingestellt wird. Dasselbe gilt auch für Gase, die aus regenerativen Quellen stammen wie etwa Biogas, das vor der Einspeisung ins Erdgasnetz auf die beschriebene Art und Weise im Brennwert oder Wobbeindex angepasst wird.

Zu den neueren Trends gehört die Zumischung von Wasserstoff zu Erdgas, wobei ersterer aus überschüssigem Strom aus regenerativen Quellen wie Photovoltaik oder Windkraft hergestellt wird. Die Zumischung zum Erdgas erlaubt es, diesen Wasserstoff mit der bestehenden Gasinfrastruktur zu speichern und zu transportieren. Gleichzeitig muss an jeder Stelle im Gasnetz jederzeit bekannt sein, wie viel Wasserstoff sich im Erdgas befindet, um die Sicherheit, die Prozessverträglichkeit und/oder die Korrektheit der Gasabrechnung sicherzustellen.

Mischtechniken gibt es in vielen Varianten, grundsätzlich werden aber immer zwei oder mehr Gasströme zu einem Ausgangsstrom vereint. Zur Kontrolle und/oder Steuerung des richtigen Mischungsverhältnisses der verschiedenen Gasströme können sowohl volumetrische als auch massenbezogene oder analytische Verfahren verwendet werden. Volumenbezogene Verfahren (Summe der Eingangsvolumenströme = Ausgangsvolumenstrom) können schnell fehlerbehaftet sein, wenn sich beim Mischprozess Druck oder Temperatur ändert. Massenbezogene Verfahren sind aufwändig und benötigen hochpräzise Messinstrumente (Waagen, Coriolismeter), während analytische Verfahren zur Messung der Gaszusammensetzung zumindest im Ausgangsstrom ebenfalls teure und mitunter nicht echtzeitfähige Apparate wie Gaschromatographen benötigen.

Ein weiteres Verfahren zur Kontrolle und/oder Steuerung des Mischungsverhältnisses ist die Mischanteilsbestimmung in Mischungen zweier Gasströme mittels Sensoren, die eine physikalische Grösse der Gasmischung messen. Von diesem Verfahren sind viele Varianten auf dem Markt erhältlich. Die meisten Ausführungen gehen davon aus, dass die Eingangsgasströme bekannt und in ihrer Zusammensetzung für den Mischprozess konstant bleiben. Die Sensoren sind von Mischprozess zu Mischprozess individuell verschieden kalibriert und liefern falsche Resultate, falls in einem der zu mischenden Gase die Zusammensetzung ändert.

In der Veröffentlichung WO 02/40992 A1 wird ein Verfahren zur Bestimmung der Gemischanteile von vier Gaskomponenten eines Gasgemisches beschrieben. In dem Verfahren wird davon ausgegangen, dass die drei physikalischen Grössen, die gemessen werden, linear von den Gemischanteilen der Komponenten abhängen. Eine vierte Gleichung ergibt sich aus der Summe der Gemischanteile, die gleich 100 % ist. Das so definierte lineare Gleichungssystem enthält zwölf Konstanten, die mittels Regressionsanalyse aus bekannten Werten für die Messgrössen bestimmt werden müssen.

In EP 0 472 131 A1 wird ein Verfahren zur Bestimmung der Konzentration einer Komponente eines aus mehreren Komponenten bestehenden Gasgemisches durch Messung der Wärmeleitfähigkeit beschrieben. Im Unterschied zum Verfahren gemäss vorliegender Erfindung werden die Gase jedoch nicht gemischt, sondern für die Bestimmung entmischt, was einen hohen Aufwand für die Entmischungsapparatur bedeuten kann.

EP2667276 offenbart eine Bestimmung der Mischanteile beim Mischen von zwei Gasen, wobei im gemischten Gas und in einem oder beiden der Gase jeweils eine physikalische Eigenschaft mit jeweils einem Sensor gemessen wird und aus den Werten der gemessenen physikalischen Eigenschaft der Mischanteil eines der Gase im gemischten Gas bestimmt wird.

Die Idee der Erfindung ist es deshalb, ein Verfahren und eine Messvorrichtung zur Bestimmung der Mischanteile beim Mischen von Gasen anzugeben, welche über die Messung physikalischer Eigenschaften der Gasströme eine kostengünstige Echtzeitbestimmung der Mischanteile der zu mischenden Gase oder von Gasbeschaffenheiten resultierend aus diesen Mischanteilen ermöglichen. Gasbeschaffenheiten können z.B. der Brennwert, der Wobbeindex oder die Methanzahl sein.

Diese Aufgabe wird durch ein Verfahren nach Anspruch 1 und durch eine Messvorrichtung nach Anspruch 13 gelöst.

Physikalische Gaseigenschaften können z.B. die Schallgeschwindigkeit, der Brechungsindex, die Diffusionskonstante, der optische Absorptionskoeffizient bei einer bestimmten Wellenlänge elektromagnetischer Strahlung, die Viskosität, die Wärmekapazität oder die Wärmeleitfähigkeit sein. Kennt man die physikalischen Gesetzmässigkeiten, wie sich eine Gaseigenschaft einer Mischung aus den Werten der zu mischenden Gase ermitteln lässt, kann das Mischungsverhältnis bestimmt werden.

Mischen zweier Gase:

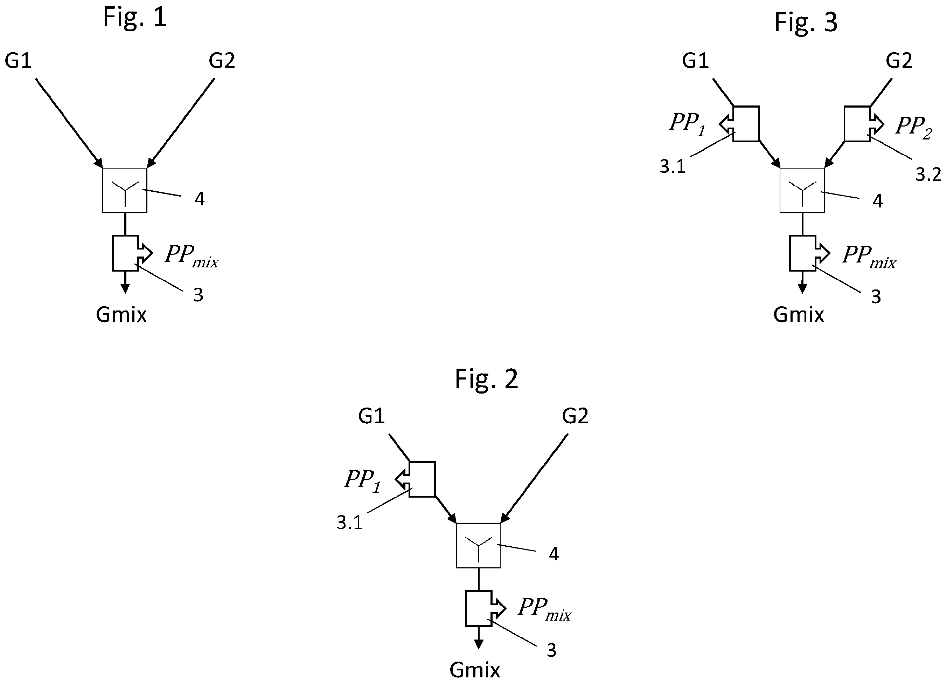

Ist die Gaszusammensetzung der zu mischenden Gase G1 und G2 bekannt, so kennt man z.B. aus normativen Berechnungsvorschriften oder Stoffdatenbanken die physikalische Eigenschaft für diese Gase, $PP_1$ und $PP_2$, basierend auf welcher die Messung vorgenommen wird. Aus dem gemessenen Wert für diese physikalische Eigenschaft im gemischten Gas, $PP_{mix}$, kann man auf die prozentuale Zusammensetzung $C_1$ und $C_2$ der Gase G1 und G2 im Mischgas schliessen (Fig. 1):

$$(1) \qquad PP_{mix} = f(PP_1, C_1, PP_2, C_2) \quad \text{und} \quad C_1 + C_2 = 100\%$$

Bei konstanter Temperatur und konstantem Druck vor und nach der Mischung ist die Funktion $f(PP_1, C_1, PP_2, C_2)$ im Falle der Dichte $\rho$ als physikalische Eigenschaft linear von der Dichte und den Mischanteilen der zu mischenden Gase abhängig:

$$(2) \qquad \rho_{mix} = f(\rho_1, C_1, \rho_2, C_2) = \frac{C_1 \cdot \rho_1 + C_2 \cdot \rho_2}{C_1 + C_2}.$$

Der Nenner in Gleichung (2) ist gleich 1, sodass mit der Bedingung $C_1 + C_2 = 1$ nach $C_1$ und $C_2$ aufgelöst werden kann:

$$(3) \qquad C_1 = \frac{\rho_{mix} - \rho_2}{\rho_1 - \rho_2} \quad \text{und} \quad C_2 = \frac{\rho_1 - \rho_{mix}}{\rho_1 - \rho_2}.$$

Da nun die Mischanteile $C_1$ und $C_2$ der beiden Gase im gemischten Gas bekannt sind, kann bei Kenntnis der Zusammensetzung von G1 und G2 die Zusammensetzung von $G_{mix}$ berechnet werden. Aus $G_{mix}$ wiederum lassen sich mittels normativen Berechnungsvorschriften oder Stoffdatenbanken eine Vielzahl von Gasbeschaffenheiten wie etwa der Brennwert oder die Methanzahl ermitteln.

In vielen Anwendungen wird einem Grundgas G1 eine weitere, bekannte Komponente G2 zugemischt, wie etwa bei der Aufbereitung von Biogas zur Einspeisung ins Erdgasnetz oder neuerdings auch bei der Zumischung von zuvor aus regenerativen Quellen erzeugtem Wasserstoff zu Erdgas. In beiden Fällen ist das sich im örtlichen Gasnetz vorzufindende Erdgas das Grundgas. Zwar sind die Schwankungen in der Zusammensetzung des Erdgases in der Regel klein, können sich aber dennoch in Form eines Fehlers in der Bestimmung der Zumischrate $C_2$ auswirken, wenn nur $PP_{mix}$ ($= \rho_{mix}$) im gemischten Gas gemessen wird. Sei $\Delta\rho_1$ die Dichteänderung zur angenommenen Dichte $\rho_1$ im Grundgas, hat das Grundgas also die Dichte $\rho_1 + \Delta\rho_1$, ergibt sich die Zumischrate $C_{2eff}$ wie folgt:

$$(4) \qquad C_{2eff} = \frac{\rho_1 + \Delta\rho_1 - \rho_{mix}}{\rho_1 + \Delta\rho_1 - \rho_2}.$$

Ohne Kenntnis der Änderung der Dichte des Grundgases G1, wäre $C_2$ anhand von Gleichung (3) mit einem entsprechenden Fehler im Vergleich zum Effektivwert in Gleichung (4) berechnet worden. Der Fehler ist umso grösser, je ähnlicher $\rho_1$, $\rho_2$, und damit auch $\rho_{mix}$ sind und beträgt im Limes $\rho_1 - \rho_2 = \rho_{mix}$ einhundert Prozent.

Eine Aufgabe der vorliegenden Erfindung besteht deshalb darin, ein Verfahren und eine Messvorrichtung zur Bestimmung der Mischanteile beim Mischen von Gasen anzugeben, welche Änderungen in der Zusammensetzung eines oder mehrerer der zu mischenden Gase berücksichtigt, und Fehler in der Bestimmung der Mischanteile vermeidet, die durch eine solche Änderung in der Zusammensetzung der zu mischenden Gase entstehen.

Diese Aufgabe wird erfindungsgemäss durch die kennzeichnenden Merkmale des Anspruchs 1 gelöst.

Mischen von drei und mehr Gasen:

Werden n ≥3 Gase miteinander gemischt, können mit der Bestimmung nur einer physikalischen Eigenschaft im gemischten Gas die prozentualen Anteile der n Gase nicht mehr eindeutig bestimmt werden.

Allgemein müssen beim Mischen von n Gasen mindestens n - 1 physikalische Eigenschaften $PP_{mix.1}$ bis $PP_{mix.n-1}$ im gemischten Gas gemessen werden, um die Anteile der n Gase im gemischten Gas zu bestimmen. Falls die n - 1 physikalischen Eigenschaften im gemischten Gas jeweils linear abhängig sind von den entsprechenden physikalischen Eigenschaften und den Mischanteilen der n Gase, ergibt sich ein lineares Gleichungssystem mit n Gleichungen und n Unbekannten:

$$(5) \quad \begin{aligned} C_1 \cdot PP_{1.1} + C_2 \cdot PP_{2.1} + \cdots + C_n \cdot PP_{n.1} &= PP_{mix.1} \\ C_1 \cdot PP_{1.2} + C_2 \cdot PP_{2.2} + \cdots + C_n \cdot PP_{n.2} &= PP_{mix.2} \\ \vdots \\ C_1 \cdot PP_{1.n-1} + C_2 \cdot PP_{2.n-1} + \cdots + C_n \cdot PP_{n.n-1} &= PP_{mix.n-1} \\ C_1 + C_2 + \cdots + C_n &= 1 \end{aligned}$$

Falls die n - 1 physikalischen Eigenschaften gegenseitig linear unabhängig voneinander sind, hat das Gleichungssystem eine eindeutige Lösung für die Mischanteile $C_1, ..., C_n$.

Der Einsatz eines zweiten Sensors im Grundgas zur Bestimmung von $C_{2eff}$:

In einer bevorzugten Ausführungsform wird beim Mischen von zwei Gasen direkt der Mischanteil ausgegeben. In diesem Fall muss auf dem Sensor die Umrechnung von gemessener, physikalischer Eigenschaft zu Mischanteil hinterlegt sein. Dies gilt umso mehr für physikalische Eigenschaften wie etwa die Wärmeleitfähigkeit $\lambda$, für welche die Mischregel nichtlinear ist und die gemessenen Signale zuerst anhand einer auf dem Sensor hinterlegten Kalibrierkurve auf ein lineares Ausgangssignal umgerechnet werden müssen. Wie aber Änderungen im Grundgas berücksichtigen, wenn die Kalibrierkurven auf dem Sensor für das Zumischen von G2 zu G1 fix hinterlegt sind?

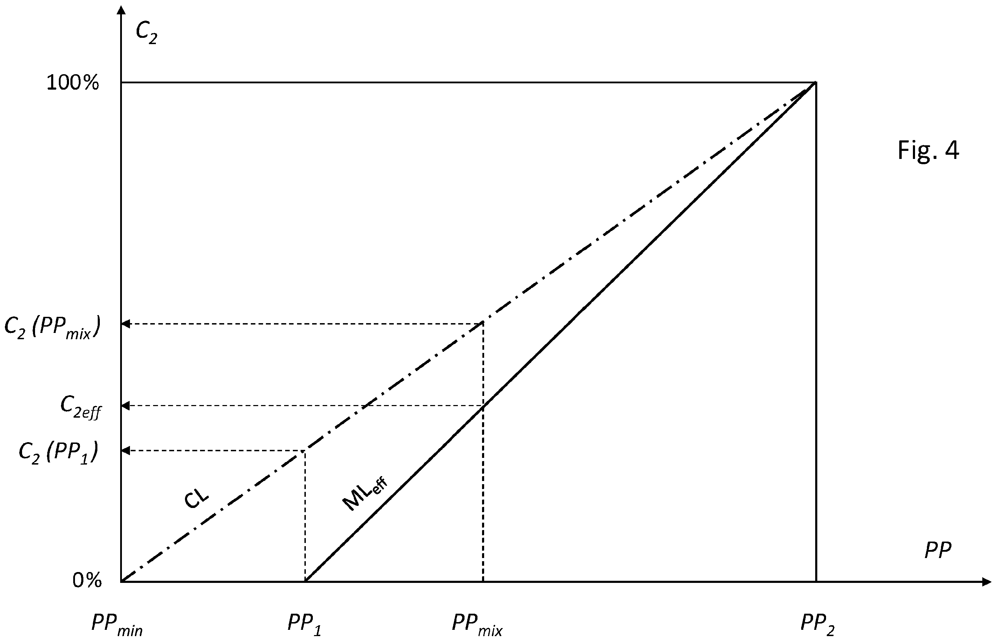

Die Idee der Erfindung ist es, ein Verfahren anzugeben, wie Änderungen in den zu mischenden Gasen bezüglich derjenigen physikalischen Eigenschaften, über die das Mischverhältnis berechnet wird, bei einem Mischvorgang berücksichtigt werden können. Zu diesem Zweck wird der Kalibrierbereich für die physikalische Eigenschaft grösser gewählt, als er für die zu mischenden Gase im Normalfall zu erwarten ist. Dieses Vorgehen ist in Fig. 4 gezeigt: $PP_2$ ist die für das Zumischgas bekannte physikalische Eigenschaft. Das andere Ende der Kalibrierskala liegt bei $PP_{min}$. $PP_{min}$ wird so gelegt, dass die physikalische Eigenschaft $PP_1$ für das Grundgas für alle zu erwartenden Änderungen im Grundgas zwischen $PP_{min}$ und $PP_2$ liegt. Dies ergibt als Kalibrierlinie CL die Strich-punktierte Figurendiagonale. Im Grundgas wird $PP_1$ mit einem zweiten, identisch kalibrierten Sensor gemessen (Fig. 2), woraus sich ein scheinbarer Mischanteil $C_2(PP_1)$ für das Zumischgas ergibt. $C_2(PP_1)$ ist ein scheinbarer Mischanteil, denn das Grundgas vor der Mischung enthält kein Zumischgas. In der Mischung wird $PP_{mix}$ ermittelt, womit ein scheinbarer Mischanteil $C_2(PP_{mix})$ für die Zumischung ausgegeben wird. Um den effektiven Mischanteil $C_{2eff}$ des Zumischgases in der Mischung zu erhalten, stellt man am besten das Gleichungssystem für die in Fig. 4 als ausgezogene Linie gezeigte, effektive Messlinie $ML_{eff}$ auf (vergleiche dazu auch Gleichung (1)):

$$(6) \quad \begin{aligned} C_{1eff} \cdot PP_1 + C_{2eff} \cdot PP_2 &= PP_{mix} \\ C_{1eff} + C_{2eff} &= 1 \end{aligned}$$

Die erste Gleichung kann auf beiden Seiten mit einem von Null verschiedenen Faktor s multipliziert werden, ohne dass sich damit etwas für die Lösung der Unbekannten $C_{1eff}$ und $C_{2eff}$ ändert. Dasselbe gilt für die zweite Gleichung bei der Multiplikation mit $s \cdot PP_{min}$ :

$$(7) \quad \begin{aligned} C_{1eff} \cdot s \cdot PP_1 + C_{2eff} \cdot s \cdot PP_2 &= s \cdot PP_{mix} \\ s \cdot PP_{min} \cdot C_{1eff} + s \cdot PP_{min} \cdot C_{2eff} &= s \cdot PP_{min} \end{aligned}$$

Ebenso kann in der ersten Gleichung des Gleichungssystems (7) auf beiden Seiten der gleiche Wert addiert oder subtrahiert werden, ohne dass sich damit etwas für die Lösung der Unbekannten $C_{1eff}$ und $C_{2eff}$ ändert. Wird demnach die linke Seite der zweiten von der linken Seite der ersten und die rechte Seite der zweiten von der rechten Seite der ersten Gleichung subtrahiert, lautet das Gleichungssystem (7) neu

(8)

$$C_{1eff} \cdot s \cdot (PP_1 - PP_{min}) + C_{2eff} \cdot s \cdot (PP_2 - PP_{min}) = s \cdot (PP_{mix} - PP_{min})$$

$$C_{1eff} + C_{2eff} = 1$$

Wenn der Faktor s := 100% / $(PP_2 - PP_{min})$ der Steigung der Kalibrierlinie CL entspricht, entsteht daraus

$$(9) \qquad C_{1eff} \cdot C_2(PP_1) + C_{2eff} \cdot C_2(PP_2) = C_2(PP_{mix})$$

$$C_{1eff} + C_{2eff} = 1$$

d.h. ein neues Gleichungssystem mit den Unbekannten $C_{1eff}$ und $C_{2eff}$ als Funktion der scheinbaren Mischanteile $C_2(PP_1), C_2(PP_2)$ und $C_2(PP_{mix})$. Dieses lässt sich einfach nach $C_{2eff}$ auflösen:

$$(10) \qquad C_{2eff} = \frac{C_2(PP_{mix}) - C_2(PP_1)}{C_2(PP_2) - C_2(PP_1)} \cdot$$

$PP_1$ und $PP_{mix}$ sind die von den beiden Sensoren im Grundgas und in der Mischung gemessenen physikalischen Eigenschaften für die jeweiligen Gase. $C_2 (PP_2)$ = 100%, wenn das Zumischgas G2 als bekannt vorausgesetzt wird.

Wenn sich das Grundgas G1 über die Zeit ändert, ändert auch die jeweilige effektive Messlinie $ML_{eff}$ in Fig. 4. Dennoch resultiert der richtige Wert für die Zumischung von Gas G2 zum Grundgas G1 mit Hilfe der scheinbaren Mischanteile $C_2(PP_1)$ und $C_2(PP_{mix})$. Die beiden Sensoren sind dabei identisch für den Wertebereich $PP_{min}$ bis $PP_2$ für die physikalische Eigenschaft PP kalibriert worden.

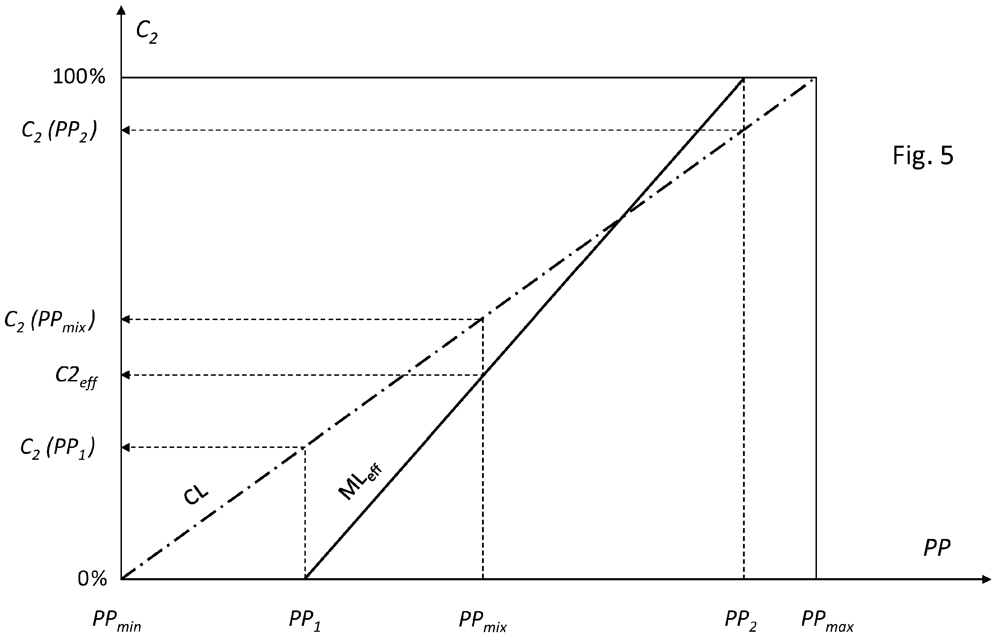

Der Einsatz eines dritten Sensors im Zumischgas zur Bestimmung von $C_{2eff}$: Die Erweiterung auf den Fall, dass auch das Zumischgas gewissen Schwankungen in der Zusammensetzung unterliegt, ist in Fig. 5 gezeigt.

Zusätzlich wird mit einem dritten Sensor im Zumischgas vor der Mischung die physikalische Eigenschaft $PP_2$ gemessen (siehe Fig. 3).

Alle drei Sensoren sind für einen Wertebereich $PP_{min}$ bis $PP_{max}$ für die physikalische Eigenschaft in gleicher Weise kalibriert, sodass $PP_1$ und $PP_2$ für alle zu erwartenden Schwankungen im Grund- und Zumischgas innerhalb dieses Wertebereichs liegen. Dies ergibt als Kalibrierlinie CL die Strich-punktierte Figurendiagonale in Fig. 5. Im Grundgas wird $PP_1$ und im Zumischgas $PP_2$ gemessen (Fig. 3), woraus sich die scheinbaren Mischanteile $C_2 (PP_1)$ für das Grundgas und $C_2(PP_2)$ für das Zumischgas ergeben. In der Mischung wird $PP_{mix}$ ermittelt, womit ein scheinbarer Mischanteil $C_2(PP_{mix})$ für die Zumischung ausgegeben wird. Um den effektiven Mischanteil $C_{2eff}$ des Zumischgases in der Mischung zu erhalten, stellt man am besten das Gleichungssystem für die in Fig. 5 als ausgezogene Linie gezeigte, effektive Messlinie $ML_{eff}$ auf.

An den Gleichungen (6) - (10) ändert sich formal nichts, ausser dass nun für die Steigung s der Kalibriergeraden

$$s := \frac{100\%}{PP_{max} - PP_{min}}$$

gesetzt werden muss.

Wiederum ist das ursprüngliche Gleichungssystem (6) mit den gemessenen, physikalischen Eigenschaften der Gase durch das Gleichungssystem (9) mit den scheinbaren Mischanteilen $C_2(PP_1)$, $C_2(PP_2)$ und $C_2(PP_{mix})$ ersetzt worden.

Bei der Bestimmung der Mischanteile werden Werte von physikalischen Eigenschaften, die nichtlinearen Mischregeln unterliegen, mit Vorteil zuerst mit Hilfe einer nichtlinearen Funktion umgerechnet, so dass die entsprechenden Funktionswerte oder Sensorsignale linearen Mischregeln unterliegen. Für physikalische Eigenschaften, die nichtlinearen Mischregeln unterliegen, sind die Fig. 4 und 5 jeweils bezüglich der sensorintern oder -extern umgerechneten Werte zu verstehen (siehe Fig. 6).

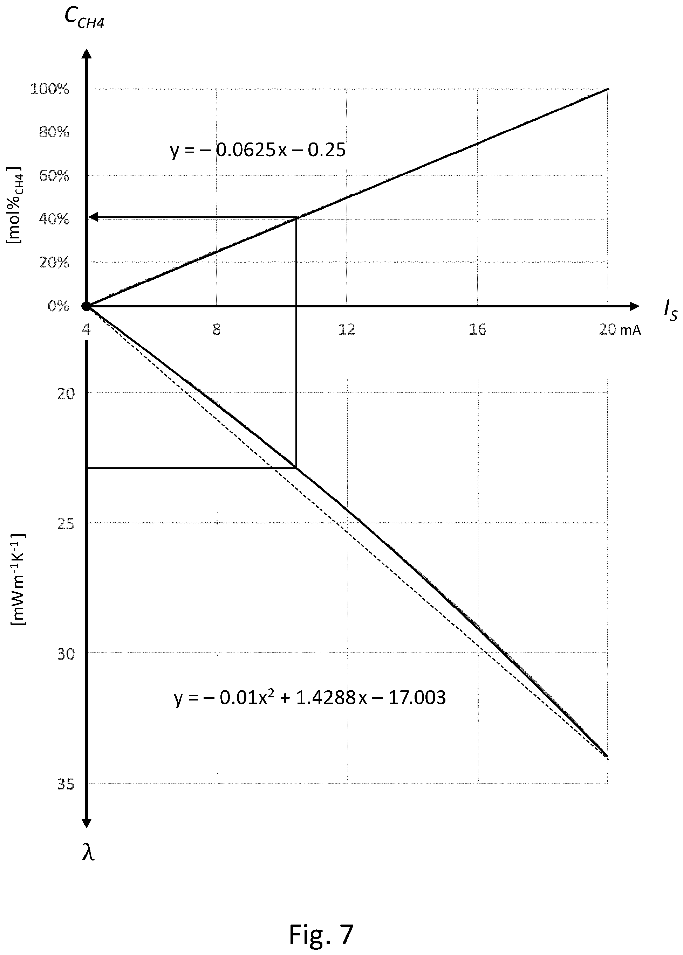

Viele Sensortypen werden mit einer analogen 4 - 20 mA Schnittstelle geliefert. Dieses Ausgangssignal sollte dann linear mit der gewünschten Zielgrösse, hier also dem Mischanteil oder scheinbaren Mischanteil des Zumischgases im Zusammenhang stehen. In Fig. 7 ist die nicht-lineare Mischregel am Beispiel der Wärmeleitfähigkeit λ für Mischungen aus Methan und Kohlendioxid (Biogas) zusammen mit der Umrechnung auf ein lineares 4 - 20 mA Ausgangssignal des Sensors gezeigt. Die Mischanteile $C_{CH4}$ sind nach der Umrechnung proportional zum Sensorausgangssignal $I_S$.

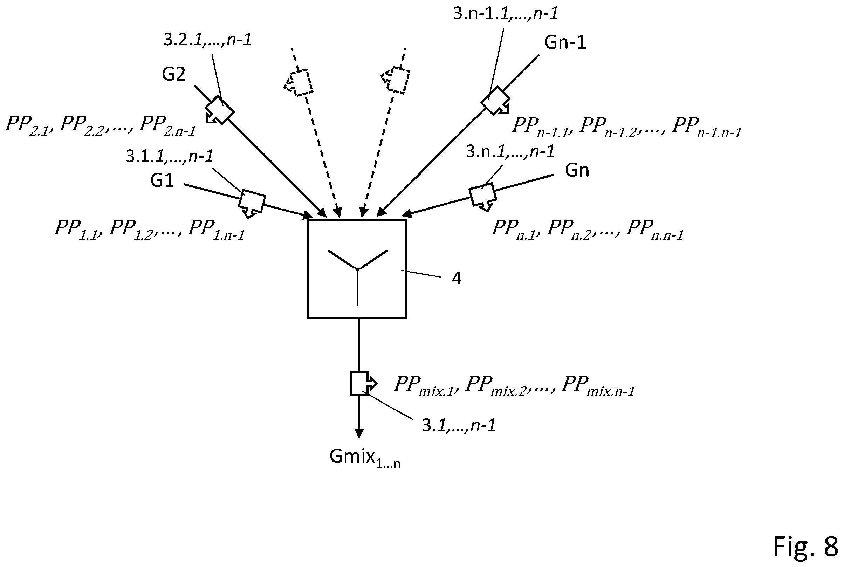

Steht lediglich eine gemessene, physikalische Eigenschaft zur Verfügung, gilt das Gesagte nur für das gleichzeitige Mischen zweier Gasströme, denn bei drei oder mehr gleichzeitigen Eingangsgasströmen kann ein Mischwert für die physikalische Gaseigenschaft im Ausgangsgasstrom mehrdeutig sein. Werden n Gase miteinander gemischt (Fig. 8), kann das oben Gesagte leicht erweitert werden auf n -1 physikalische Grössen, die mit n -1 bis (n - 1) · (n + 1) = $n^2$ -1 Sensoren gemessen werden können. Das Gleichungssystem (6) wird auf n Gleichungen erweitert und lautet:

(11)

$$C_{1eff} \cdot PP_{1.1} + C_{2eff} \cdot PP_{2.1} + \cdots + C_{neff} \cdot PP_{n.1} = PP_{mix.1}$$
$$C_{1eff} \cdot PP_{1.2} + C_{2eff} \cdot PP_{2.2} + \cdots + C_{neff} \cdot PP_{n.2} = PP_{mix.2}$$
$$\vdots$$
$$C_{1eff} \cdot PP_{1.n-1} + C_{2eff} \cdot PP_{2.n-1} + \cdots + C_{neff} \cdot PP_{n.n-1} = PP_{mix.n-1}$$
$$C_{1eff} + C_{2eff} + \cdots + C_{neff} = 1$$

mit den Unbekannten $C_{1eff}$ bis $C_{neff}$.

Wiederum können die ersten n -1 Gleichungen auf beiden Seiten mit der Steigung $s_i$ = 100% / $(PP_{maxi} - PP_{mini})$ der zur physikalischen Eigenschaft $PP_i$ gehörenden Kalibriergeraden multipliziert werden ($i = 1$ bis n -1), ohne dass sich damit etwas für die Lösung der Unbekannten $C_{1eff}$ bis $C_{neff}$ ändert. Nun wird analog zum Gleichungssystem (8) je auf der linken und rechten Seite der ersten n -1 Gleichungen die mit $s_i \cdot PP_{mini}$ multiplizierte linke bzw. rechte Seite der letzten Gleichung subtrahiert, woraus analog zum Gleichungssystem (8) bzw. (9) ein neues Gleichungssystem für die Unbekannten $C_{1eff}$ bis $C_{neff}$ als Funktion der (n-1)·(n+1) = $n^2$-1 scheinbaren Mischanteile $C_{1.1}(PP_{1.1})$, $C_{2.1}(PP_{2.1})$ bis $C_{n.n-1}$ $(PP_{n.n-1})$ entsteht:

(12)

$$C_{1eff} \cdot C_{1.1}(PP_{1.1}) + C_{2eff} \cdot C_{2.1}(PP_{2.1}) + \cdots + C_{neff} \cdot C_{n.1}(PP_{n.1}) = C_{mix.1}(PP_{mix.1})$$
$$C_{1eff} \cdot C_{1.2}(PP_{1.2}) + C_{2eff} \cdot C_{2.2}(PP_{2.2}) + \cdots + C_{neff} \cdot C_{n.2}(PP_{n.2}) = C_{mix.2}(PP_{mix.2})$$
$$\vdots$$
$$C_{1eff} \cdot C_{1.n-1}(PP_{1.n-1}) + C_{2eff} \cdot C_{2.n-1}(PP_{2.n-1}) + \cdots + C_{neff} \cdot C_{n.n-1}(PP_{n.n-1})$$
$$= C_{mix.n-1}(PP_{mix.n-1})$$
$$C_{1eff} + C_{2eff} + \cdots + C_{neff} = 1$$

Zur Lösung dieses Gleichungssystem kann beispielsweise bis n = 3 mit Determinanten gerechnet werden. Ab n = 4 empfiehlt es sich, andere numerische Verfahren wie etwa das Eliminationsverfahren von Gauss anzuwenden.

Wird nur in m der n Zumischgase mit Schwankungen der Gaszusammensetzung gerechnet, muss folglich nur mit m·(n -1) Sensoren der scheinbare Mischanteil dieser m Gase gemessen werden. Die (n - m)·(n - 1) physikalischen Eigenschaften $PP_{m+1.1}$, $PP_{m+1.2}$ ... $PP_{n.n-1}$ der restlichen n - m Gase sind anhand der Zusammensetzung dieser Gase bekannt, woraus sich die scheinbaren Mischanteile $C_{m+1.1}(PP_{m+1.1})$, $C_{m+1.2}(PP_{m+1.2})$ ... $C_{n.n-1}(PP_{n.n-1})$ im Gleichungssystem (12) aus der Kalibrierkurve für die scheinbaren Mischanteile (Strich-punktierte Figurendiagonale CL in Fig. 5) berechnen lassen.

In dem Verfahren zur Bestimmung der Mischanteile beim Mischen von n = 2 oder 3 oder 4 oder mehr Gasen gemäss vorliegender Erfindung werden im gemischten Gas und/oder in einem oder mehreren der n Gase jeweils n -1 physikalische Eigenschaften gemessen, wobei die n -1 physikalischen Eigenschaften zum Beispiel mit zwei oder drei oder mehr Sensoren gemessen werden können, und wobei aus den Werten der gemessenen physikalischen Eigenschaft oder Eigenschaften die Mischanteile der n Gase im gemischten Gas bestimmt werden.

Mit Vorteil werden mindestens eine physikalische Eigenschaft sowohl im gemischten Gas als auch in mindestens einem der n Gase oder mindestens eine oder mindestens zwei physikalische Eigenschaften im gemischten Gas gemessen.

In einer vorteilhaften Ausführungsform wird für diejenigen physikalischen Eigenschaften, für die eine lineare Beziehung zwischen der im gemischten Gas gemessenen physikalischen Eigenschaft einerseits und der entsprechenden physikalischen Eigenschaft und den Mischanteilen der n Gase andererseits besteht oder für die die Abweichung von der Linearität im Bereich, in dem gemessen wird, innerhalb eines Toleranzbereichs von 0.2% oder 0.5% oder 1% liegt, die entsprechende lineare Beziehung für die Bestimmung der Mischanteile verwendet.

Für diejenigen physikalischen Eigenschaften, für die keine lineare Beziehung zwischen der im gemischten Gas gemessenen physikalischen Eigenschaft einerseits und der entsprechenden physikalischen Eigenschaft und den Mischanteilen der n Gase andererseits besteht und/oder für die die Abweichung von der Linearität im Bereich, in dem gemessen wird, ausserhalb eines Toleranzbereichs von 0.2% oder 0.5% oder 1% liegt, werden die Werte der betreffenden physikalischen Eigenschaft mit Vorteil durch Funktionswerte ersetzt, die durch Einsetzen ersterer in eine nichtlineare Funktion bestimmt werden, beispielsweise durch Einsetzen in ein Polynom zweiten, dritten oder höheren Grades, so dass der Funktionswert der im gemischten Gas gemessenen physikalischen Eigenschaft linear von den Funktionswerten der betreffenden physikalischen Eigenschaft und den Mischanteilen der n Gase abhängt, und für die Bestimmung der Mischanteile jeweils die lineare Beziehung zwischen dem Funktionswert der im gemischten Gas gemessenen physikalischen Eigenschaft und den Funktionswerten der entsprechenden physikalischen Eigenschaft und den Mischanteilen der n Gase verwendet.

In einer weiteren vorteilhaften Ausführungsform werden für diejenigen physikalischen Eigenschaften, für die eine lineare Beziehung zwischen der im gemischten Gas gemessenen physikalischen Eigenschaft einerseits und der entsprechenden physikalischen Eigenschaft und den Mischanteilen der n Gase andererseits verwendet wird, die im gemischten Gas gemessene physikalische Eigenschaft als Linearkombination der entsprechenden physikalischen Eigenschaft der n Gase betrachtet und für diejenigen physikalischen Eigenschaften, für die keine lineare Beziehung zwischen der im gemischten Gas gemessenen physikalischen Eigenschaft einerseits und der entsprechenden physikalischen Eigenschaft und den Mischanteilen der n Gase andererseits verwendet wird, der Funktionswert, der aus dem Wert der im gemischten Gas gemessenen physikalischen Eigenschaft bestimmt wird, als Linearkombination der aus den Werten der entsprechenden physikalischen Eigenschaft der n Gase bestimmten Funktionswerte betrachtet, wobei vorteilhafterweise für alle n - 1 physikalischen Eigenschaften die Mischanteile den Koeffizienten der Linearkombinationen entsprechen, und aus den n-1 Linearkombinationen und aus der Kenntnis, dass die Summe der Mischanteile gleich eins oder 100 % ist, die Mischanteile bestimmt werden können.

Typisch werden für diejenigen der n Gase, in denen keine oder nicht alle der n - 1 physikalischen Eigenschaften gemessen werden, bei der Bestimmung der Mischanteile für die nicht gemessenen physikalischen Eigenschaften oder für die aus denselben bestimmten Funktionswerte vorgegebene Werte eingesetzt, wobei die vorgegebenen Werte beispielsweise aus der Zusammensetzung der betreffenden Gase bestimmt werden können.

Als physikalische Eigenschaft oder Eigenschaften können beispielsweise die Wärmeleitfähigkeit, die Wärmekapazität, die Dichte, die Viskosität, die Schallgeschwindigkeit, der Diffusionskoeffizient, der Brechungsindex, die Dielektrizitätskonstante und/oder der optische Absorptionskoeffizient bei einer bestimmten Wellenlänge elektromagnetischer Strahlung gemessen werden.

In einer bevorzugten Ausführungsform werden die n - 1 physikalischen Eigenschaften im gemischten Gas mit Sensoren gemessen, die jeweils für die betreffende physikalische Eigenschaft eine Kalibrierkurve für die Bestimmung eines scheinbaren Mischanteils enthalten, d.h. eines Mischanteils für eine Gaszusammensetzung, die nicht der effektiv vorhandenen Gaszusammensetzung zu entsprechen braucht, und aus den derart bestimmten scheinbaren Mischanteilen für jedes der n Gase der effektive Mischanteil im gemischten Gas bestimmt wird.

Bei Bedarf können die n - 1 physikalischen Eigenschaften in einem oder mehreren der n Gase mit Sensoren gemessen werden, die jeweils für die betreffende physikalische Eigenschaft eine Kalibrierkurve für die Bestimmung eines scheinbaren Mischanteils enthalten, wobei diese identisch ist mit der Kalibrierkurve in dem für die Messung der betreffenden physikalischen Eigenschaft im gemischten Gas verwendeten Sensor, und aus den derart bestimmten scheinbaren Mischanteilen für jedes der n Gase der effektive Mischanteil im gemischten Gas bestimmt wird.

Mit Vorteil werden für diejenigen der n Gase, in denen keine oder nicht alle der n -1 physikalischen Eigenschaften gemessen werden, für die scheinbaren Mischanteile vorgegebene Werte eingesetzt, wobei die vorgegebenen Werte beispielsweise aus der Zusammensetzung der betreffenden Gase bestimmt werden.

In einer vorteilhaften Ausführungsform werden Werte von physikalischen Eigenschaften, die nichtlinearen Mischregeln unterliegen, zuerst mit Hilfe einer nichtlinearen Funktion umgerechnet, so dass die Funktionswerte oder entsprechenden Sensorsignale linearen Mischregeln unterliegen.

Eine physikalische Eigenschaft $PP_i$ unterliegt einer linearen Mischregel, falls

$$PP_{mix.i} = C_1 \cdot PP_{1.i} + C_2 \cdot PP_{2.i} + \cdots + C_n \cdot PP_{n.i} \, ,$$

wobei $PP_{mix.i}$ und $PP_{1,i} \ldots PP_{n,i}$ der physikalischen Eigenschaft im gemischten Gas und in den n Gasen und $C_1 \ldots C_n$ den Mischanteilen der n Gase entsprechen.

Das bedeutet andererseits, dass eine physikalische Eigenschaft $PP_i$ einer nichtlinearen Mischregel unterliegt, falls

$$PP_{mix.i} \neq C_1 \cdot PP_{1.i} + C_2 \cdot PP_{2.i} + \cdots + C_n \cdot PP_{n.i} .$$

In einer vorteilhaften Ausführungsvariante werden $n \geq 3$ Gase sequentiell gemischt, indem zuerst zwei Gase gemischt werden und danach das Mischgas in einem weiteren Zweigasmischprozess mit einem weiteren Gas gemischt wird und das Mischen des zuletzt gemischten Mischgases mit einem weiteren Gas wiederholt wird, bis alle n Gase miteinander gemischt sind, wobei alle Sensoren identisch für dieselbe physikalische Eigenschaft kalibriert sind.

In einer weiteren vorteilhaften Ausführungsvariante werden $n \geq 4$ Gase jeweils zu zweien in einer ersten und danach die jeweiligen Mischgase in einer oder mehreren weiteren Kaskaden zu zweien gemischt, bis alle n Gase gemischt sind, wobei alle Sensoren identisch für dieselbe physikalische Eigenschaft kalibriert sind.

In einer weiteren vorteilhaften Ausführungsvariante erfolgt das Mischen der $n \geq 4$ Gase in einer Kombination von sequentiellem und kaskadiertem Mischen, wobei alle Sensoren identisch für dieselbe physikalische Eigenschaft kalibriert sind.

Weiter umfasst die Erfindung eine Messvorrichtung zur Bestimmung der Mischanteile beim Mischen von n = 2, 3, 4 oder mehr Gasen, welche einen oder mehrere Sensoren zum Messen von n -1 physikalischen Eigenschaften im gemischten und/oder in einem oder mehreren der n Gase und eine Auswerteeinheit umfasst, die mit den Sensoren verbunden ist, und die zur Ausführung eines Verfahrens gemäss vorliegender Erfindung oder einer der oben beschriebenen Ausführungsformen oder -varianten eingerichtet ist.

Die Auswerteeinheit kann beispielsweise in einer Rechen- oder Steuereinheit einer Mischvorrichtung ausgebildet oder in die Rechen- oder Steuereinheit integriert sein. Weiter können die Messvorrichtung und/oder der eine Sensor oder einer oder mehrere der Sensoren als mobil einsetzbare Geräte ausgebildet sein.

In einer weiteren vorteilhaften Ausführungsform des Verfahrens zur Mischanteilsbestimmung beim Mischen von n = 2, 3, 4 und mehr Gasen gemäss vorliegender Erfindung werden mit einem oder mehreren Sensoren n -1 physikalische Eigenschaften im gemischten Gas gemessen, wobei für jede physikalische Eigenschaft eine entsprechende Kalibrierkurve für die Bestimmung einer scheinbaren Konzentration abgelegt ist, z.B. in dem oder den entsprechenden Sensoren, und aus den Messwerten für die n-1 physikalischen Eigenschaften mit Hilfe der jeweiligen Kalibrierkurve scheinbare Konzentrationen berechnet und aus den scheinbaren Konzentrationen die effektiven Konzentrationen der n Gase im gemischten Gas bestimmt werden.

Bei Bedarf können zusätzlich in $1 \leq m \leq n$ der zu mischenden Gase die n - 1 physikalischen Eigenschaften gemessen werden, wobei für jedes der m zu mischenden Gase aus den Messwerten für die n - 1 physikalischen Eigenschaften mit Hilfe der jeweiligen Kalibrierkurve scheinbare Konzentrationen bestimmt werden, und aus den scheinbaren Konzentrationen der m zu mischenden Gase und derjenigen des gemischten Gases die effektiven Konzentrationen der n Gase im gemischten Gas bestimmt werden.

In einer vorteilhaften Ausführungsvariante, in der n = 2 und m = 0 ist, werden aus der bekannten physikalischen Eigenschaft $PP_1$ und $PP_2$ von Gas 1 und 2 die scheinbaren Mischanteile von Gas 1 und 2 berechnet und zusammen mit dem aus der gemessenen physikalischen Eigenschaft $PP_{mix}$ bestimmten scheinbaren Mischanteil des Mischgases die effektive Konzentration von Gas 2 und im Komplement zu 100% die effektive Konzentration von Gas 1 im Mischgas bestimmt.

In einer weiteren vorteilhaften Ausführungsvariante, in der n = 2 und m = 1 ist, werden aus der bekannten physikalischen Eigenschaft $PP_2$ von Gas 2 der scheinbare Mischanteil von Gas 2 berechnet und zusammen mit den aus der gemessenen physikalischen Eigenschaft $PP_1$ und $PP_{mix}$ bestimmten scheinbaren Mischanteilen von Gas 1 und des Mischgases die effektive Konzentration von Gas 2 und im Komplement zu 100% die effektive Konzentration von Gas 1 im Mischgas bestimmt.

In einer anderen vorteilhaften Ausführungsvariante, in der n = 2 und m = 2 ist, werden mit den aus den gemessenen physikalischen Eigenschaft $PP_1$, $PP_2$ und $PP_{mix}$ bestimmten scheinbaren Mischanteilen von Gas 1, Gas 2 und des Mischgases die effektive Konzentration von Gas 2 und im Komplement zu 100% die effektive Konzentration von Gas 1 im Mischgas bestimmt.

Die physikalischen Eigenschaften können z.B. die Wärmeleitfähigkeit, die Wärmekapazität, die Dichte, die Viskosität, die Schallgeschwindigkeit der Diffusionskoeffizient, der Brechungsindex, die Dielektrizitätskonstante und/oder der optische Absorptionskoeffizient bei einer bestimmten Wellenlänge elektromagnetischer Strahlung sein.

Falls die physikalische Eigenschaft die Wärmeleitfähigkeit ist, werden der oder die Sensoren mit Vorteil für den Wertebereich von Krypton ($\lambda=9.5\cdot10^{-3}$ W m$^{-1}$ K$^{-1}$ bei 25 °C und 1013 mbar) bis Wasserstoff ($\lambda = 0.186$ W m$^{-1}$ K$^{-1}$ bei 25 °C und 1013 mbar) kalibriert.

Zweckmässigerweise werden physikalische Eigenschaften, die nichtlinearen Mischregeln unterliegen, gemäss dieser nichtlinearen Mischregel zuerst auf ein lineares Sensorsignal umgerechnet, beispielsweise mit Hilfe einer nichtlinearen Funktion, so dass die Funktionswerte oder entsprechenden Sensorsignale linearen Mischregeln unterliegen

In einer vorteilhaften Ausführungsvariante werden in sequentieller Manier zuerst zwei Gase gemischt und danach das Mischgas in einem weiteren Zweigasmischprozess mit einem dritten Gas und dieses Mischgas wiederum mit einem vierten Gas gemischt, bis $n \geq 3$ Gase miteinander gemischt sind.

In einer weiteren vorteilhaften Ausführungsvariante werden jeweils 4, 8 oder 12 der $n \geq 4$ Gase in einer ersten und danach die jeweiligen Mischgase in einer zweiten Kaskade zu zweien gemischt, bis alle n Gase gemischt sind.

In einer weiteren vorteilhaften Ausführungsvariante erfolgt die Mischung der $n \geq 4$ Gase in einer Kombination von sequentiellem und kaskadiertem Mischen.

Weiter umfasst die Erfindung eine Mischvorrichtung, welche eine Gasmischeinheit mit mindestens zwei Gaszuleitungen und einer Gasausgangsleitung sowie eine Auswerteeinheit umfasst, wobei in der Gasausgangsleitung und bei Bedarf jeweils in einer oder mehreren Gaszuleitungen Sensoren für $n-1$ physikalische Eigenschaften angeordnet sind, und wobei die Sensoren mit der Auswerteeinheit verbunden sind, welche zur Ausführung eines Verfahrens gemäss vorliegender Erfindung oder einer der oben beschriebenen Ausführungsformen oder -varianten eingerichtet ist.

In einer vorteilhaften Ausführungsvariante ist die Auswerteeinheit in einer Rechen- oder Steuereinheit der Mischvorrichtung ausgebildet.

Mit Vorteil sind der Sensor oder die Sensoren für eine bestimmte physikalische Eigenschaft identisch auf einen Messbereich kalibriert, der grösser als der von den n Gasen für diese physikalische Eigenschaft zu erwartende Wertebereich ist.

In einer bevorzugten Ausführungsform der Mischvorrichtung zum Mischen von $n \geq 3$ Gasen enthält die Mischvorrichtung $n-1$ sequentiell angeordnete Gasmischeinheiten mit jeweils zwei Gaszuleitungen und einer Gasausgangsleitung, wobei die Gasausgangsleitung einer Gasmischeinheit mit Ausnahme der Gasausgangsleitung der letzten Gasmischeinheit jeweils mit einer Gaszuleitung der nachfolgenden Gasmischeinheit verbunden ist.

In einer weiteren bevorzugten Ausführungsform der Mischvorrichtung zum Mischen von $2^n$ Gasen, wobei $n \geq 2$ ist, enthält die Mischvorrichtung $2^n-1$ in einer binären Baumstruktur angeordnete Gasmischeinheiten mit jeweils zwei Gaszuleitungen und einer Gasausgangsleitung.

Die meisten bekannten Verfahren zur Mischanteilsbestimmung von Gasmischungen mittels Sensoren, die eine oder mehrere physikalische Grössen der Gasmischung messen, gehen davon aus, dass die Eingangsgasströme bekannt und in ihrer Zusammensetzung für den Mischprozess konstant bleiben. Im Gegensatz zum Verfahren und der Mischvorrichtung gemäss vorliegender Erfindung sind die Sensoren von Mischprozess zu Mischprozess individuell verschieden kalibriert und liefern falsche Resultate, falls dennoch einmal eines der zu mischenden Gase die Zusammensetzung ändert. Das Verfahren und die Mischvorrichtung gemäss vorliegender Erfindung haben dagegen den Vorteil, dass sie die korrekte Bestimmung der Mischanteile ermöglichen, selbst wenn sich die Zusammensetzung der zu mischenden Gase ändert.

Das Verfahren und die Messvorrichtung gemäss vorliegender Erfindung haben gegenüber dem in WO 02/40992 A1 beschriebenen Verfahren zur Bestimmung der Gemischanteile eines Gasgemisches aus vier Komponenten den Vorteil, dass im erfindungsgemässen Verfahren physikalische Eigenschaften, die nichtlinearen Mischregeln unterliegen, zuerst umgerechnet werden, so dass die umgerechneten Werte linearen Mischregeln unterliegen. Damit ist es möglich, die Mischanteile genauer zu bestimmen. In dem Verfahren aus WO 02/40992 wird dagegen davon ausgegangen, dass die drei physikalischen Grössen, die gemessen werden, linear von den Gemischanteilen abhängen, was z.B. für die Wärmeleitfähigkeit nicht der Fall ist.

Das lineare Gleichungssystem zur Bestimmung der Gemischanteile in WO 02/40992 A1, Seite 5, enthält zwölf Konstanten, die mittels Regressionsanalyse aus bekannten Werten für die Messgrössen bestimmt werden müssen. Demgegenüber hat das erfindungsgemässe Verfahren den Vorteil, dass die Gleichungssysteme (5) und (12) zur Bestimmung der Mischanteile ausschliesslich Konstanten enthalten, die durch die aktuelle Messung der physikalischen Eigenschaften gegeben oder aus Literatur oder Datenbanken bekannt sind. Im Gegensatz zum Stand der Technik ist deshalb keine zusätzliche Eichung zur Bestimmung der Konstanten erforderlich.

Die Erfindung wird im Folgenden anhand der Zeichnungen näher erläutert. Es zeigen:

Fig. 1    ein Beispiel für das Mischen zweier Gase, wobei nur im gemischten Gas die physikalischen Eigenschaft $PP_{mix}$ gemessen wird (n = 2, m = 0),

Fig. 2    ein zweites Beispiel für das Mischen zweier Gase, wobei im Grundgas und im gemischten Gas die physikalischen Eigenschaft $PP_1$ und $PP_{mix}$ gemessen werden (n = 2, m = 1),

Fig. 3    ein drittes Beispiel für das Mischen zweier Gase, wobei im Grundgas, im Zumischgas und im gemischten Gas die physikalische Eigenschaft $PP_1$, $PP_2$ und $PP_{mix}$ gemessen werden (n=2, m=2),

Fig. 4    ein Beispiel einer Kalibrier- und Messkurve der physikalischen Eigenschaft $PP$ beim Mischen eines Grundgases G1 mit dem Zumischgas G2 für den Fall von Schwankungen der physikalischen Eigenschaft $PP_1$ im

Grundgas (n = 2, m = 1),

Fig. 5    ein zweites Beispiel einer Kalibrier- und Messkurve der physikalischen Eigenschaft $PP$ beim Mischen eines Grundgases G1 mit dem Zumischgas G2 für den Fall von Schwankungen der physikalischen Eigenschaft $PP_1$ im Grundgas und $PP_2$ im Zumischgas (n=2, m=2),

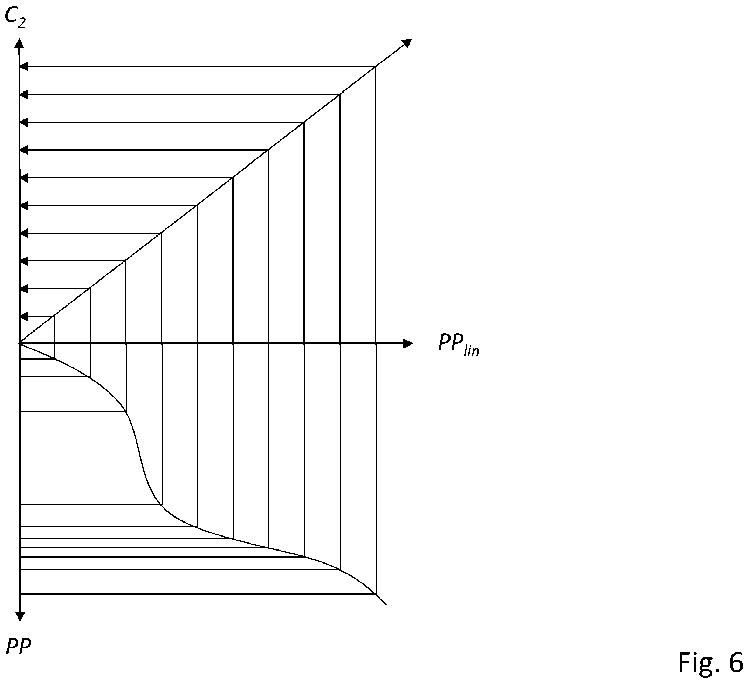

Fig. 6    ein Beispiel für eine Umrechnung des Sensorsignals auf eine lineare Kennlinie bei nicht-linearem Mischverhalten der physikalischen Eigenschaft $PP$,

Fig. 7    ein Beispiel für eine Umrechnung des Sensorsignals auf eine bezüglich des Mischanteils lineare Kennlinie bei nichtlinearem Mischverhalten für die physikalische Eigenschaft "Wärmeleitfähigkeit" für Methan/Kohlendioxidmischungen,

Fig. 8    ein Beispiel für das gleichzeitige Mischen von n Gasen mit Sensoren für n - 1 physikalische Eigenschaften $PP_i$, $(i = 1, \dots, n - 1)$,

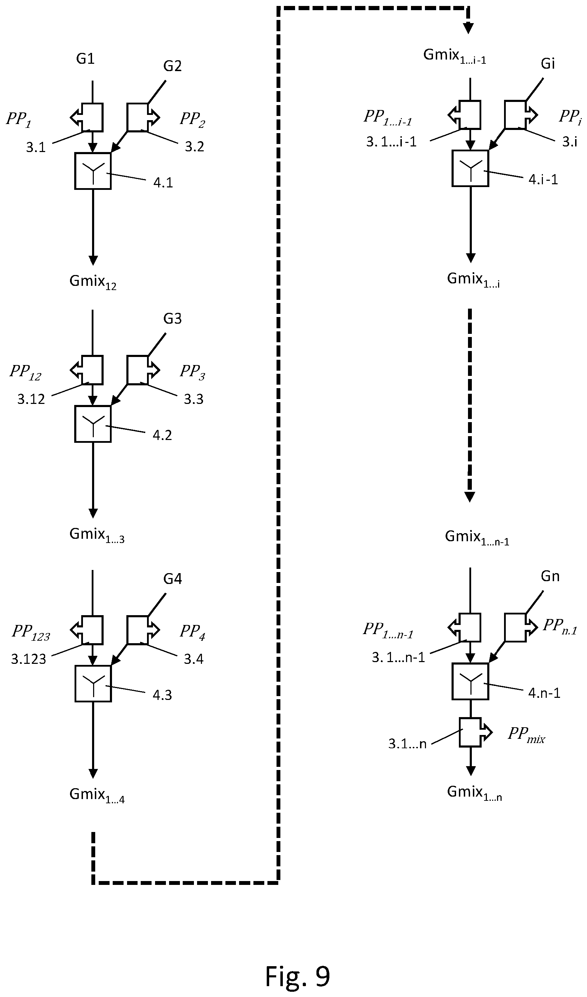

Fig. 9    ein Beispiel für das sequentielle Mischen von n Gasen mit Sensoren für eine physikalische Eigenschaft $PP$,

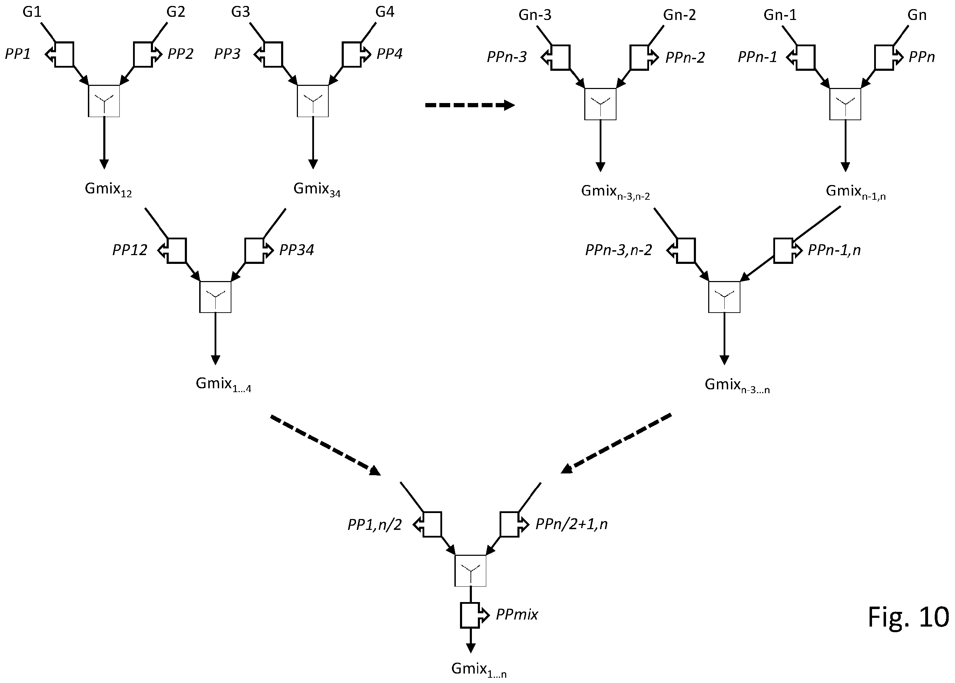

Fig. 10    ein Beispiel für das kaskadierte Mischen von $2^n$ Gasen mit Sensoren für eine physikalische Eigenschaft $PP$, und

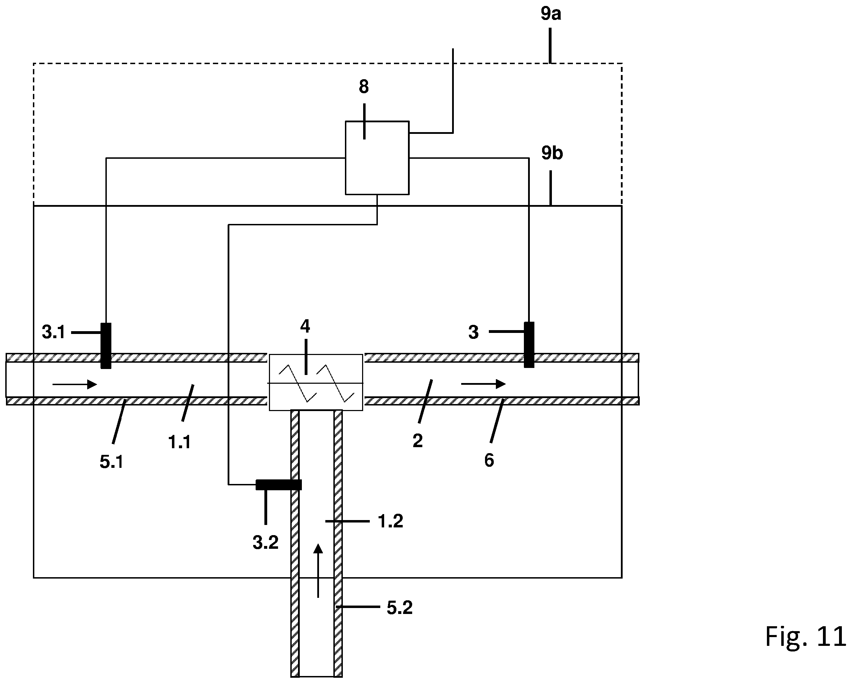

Fig. 11    ein Beispiel einer Mischvorrichtung für das Mischen zweier Gase zusammen mit einem Ausführungsbeispiel einer Messvorrichtung gemäss vorliegender Erfindung.

Die Figuren 1, 2 und 3 zeigen Beispiele für das Mischen zweier Gase G1 und G2, die einer Gasmischeinheit 4 zugeführt werden. Im gemischten Gas Gmix wird jeweils mit einem Sensor 3 die physikalische Eigenschaft $PP_{mix}$ gemessen. In dem in Fig. 1 gezeigten Beispiel wird für die Bestimmung der Mischanteile davon ausgegangen, dass die Zusammensetzung der Gase G1 und G2 bekannt ist.

Ist nur die Zusammensetzung des Zumischgases G2 bekannt, während die Zusammensetzung des Grundgases G1 Schwankungen unterliegt, wird im Verfahren gemäss vorliegender Erfindung die physikalische Eigenschaft $PP_1$ zusätzlich, wie in Fig. 2 gezeigt, mit einem Sensor 3.1 auch im Grundgas gemessen.

In dem in Fig. 3 gezeigten Beispiel wird für die Bestimmung der Mischanteile davon ausgegangen, dass die Zusammensetzungen des Grundgases G1 und des Zumischgases G2 Schwankungen unterliegen. In diesem Fall wird die physikalische Eigenschaft $PP_1$, $PP_2$ zusätzlich mit jeweils einem Sensor 3.1 und 3.2 sowohl im Grundgas als auch im Zumischgas gemessen.

In einem ersten Ausführungsbeispiel des Verfahrens gemäss vorliegender Erfindung wird die Bestimmung der Mischanteile am Beispiel der Biogaskonditionierung gezeigt. Bei der Konditionierung wird Methan aus der Biogasproduktion mit $CO_2$ und Propan gemischt.

Zur Bestimmung der Mischanteile werden im gemischten Gas die Dichte und die Viskosität gemessen. Die Werte für Methan, $CO_2$ und Propan werden nicht gemessen, sondern als bekannt vorausgesetzt.

| Gas/ physikalische Eigenschaft 25 °C, 1013.25 mbar | Methan | $CO_2$ | Propan | Messung konditioniertes Biomethan |
|---|---|---|---|---|
| Dichte [kg/m$^3$] | 0.657 | 1.808 | 1.832 | 0.876 |
| Viskosität [µPa·s] | 11.1 | 14.9 | 8.12 | 11.2 |

Einsetzen der Dichte und Viskosität in das Gleichungssystem (5) ergibt:

$$C_1 \cdot \rho_1 + C_2 \cdot \rho_2 + C_3 \cdot \rho_3 = \rho_{mix}$$

$$C_1 \cdot \eta_1 + C_2 \cdot \eta_2 + C_3 \cdot \eta_3 = \eta_{mix}$$

$$C_1 + C_2 + C_3 = 1$$

oder, falls die Mischanteile in mol% ausgedrückt werden,

$$C_1 \cdot \rho_1 + C_2 \cdot \rho_2 + C_3 \cdot \rho_3 = \rho_{mix} \cdot 100 \ mol\%$$

$$C_1 \cdot \eta_1 + C_2 \cdot \eta_2 + C_3 \cdot \eta_3 = \eta_{mix} \cdot 100 \ mol\%$$

$$C_1 + C_2 + C_3 = 100 \ mol\%$$

Mit den Werten aus dem Ausführungsbeispiel und durch Weglassen der Masseinheiten erhält man

$$C_1 \cdot 0.657 + C_2 \cdot 1.808 + C_3 \cdot 1.832 = 0.876 \cdot 100 \ mol\%$$

$$C_1 \cdot 11.1 + C_2 \cdot 14.9 + C_3 \cdot 8.12 = 11.2 \cdot 100 \ mol\%$$

$$C_1 + C_2 + C_3 = 100 \ mol\%$$

Die Lösung des obigen Gleichungssystems kann beispielsweise mittels der Cramer'schen Regel (Determinantenmethode) ermittelt werden. Als Lösung erhält man $C_1$= 81.1 mol% (Methan), $C_2$ = 9.8 mol% ($CO_2$) und $C_3$ = 9.1 mol% (Propan). Effektiv stammen die Messwerte von einem konditionierten Biomethan mit der Zusammensetzung $C_1$ = 81 mol%, $C_2$ = 10 mol% und $C_3$ = 9 mol%.

Ein zweites Ausführungsbeispiel des Verfahrens gemäss vorliegender Erfindung wird im Folgenden anhand des Zumischens von Wasserstoff in Erdgastransportleitungen beschrieben. Da sich die Erdgaszusammensetzung ständig ändern kann, zu Beispiel weil das Erdgas abhängig von Verfügbarkeit und Preis aus verschiedenen Quellen stammt, ist es vorteilhaft, sowohl das Erdgas vor als auch das Mischgas nach der Zumischung von Wasserstoff zu messen, um die Zumischmenge von letzterem zu bestimmen. Da die Eigenschaften von reinem Wasserstoff bekannt sind, braucht es für letzteren keinen zusätzlichen Sensor.

Im zweiten Ausführungsbeispiel wird als physikalische Eigenschaft die Wärmeleitfähigkeit λ gemessen, wobei zwei Sensoren benötigt werden, deren Messbereich Wärmeleitfähigkeiten von λ = 25.84 mWm$^{-1}$K$^{-1}$ (Stickstoff, $N_2$) bis λ = 185.8 mW m$^{-1}$ K$^{-1}$ (Wasserstoff, $H_2$) umfasst. Stickstoff entspricht einer gemessenen Wasserstoffkonzentration von 0 mol%, während reiner Wasserstoff 100 mol% bedeutet. Die Wärmeleitfähigkeit einer Mischung aus $H_2$ und $N_2$ ist jedoch nicht proportional zur molaren Konzentration von $H_2$ in $N_2$. So hat eine 50%/50%-Mischung linear gerechnet eine Wärmeleitfähigkeit von 105.8 mW m$^{-1}$K$^{-1}$, während der physikalische Wert bei 76.42 mW m$^{-1}$ K$^{-1}$ liegt. Würde man letzteren in das Gleichungssystem (6) und die entsprechende Lösung, Gleichung (10) einsetzen, käme eine Mischung von 68.4%/31.6% $N_2$/$H_2$ heraus. Folglich muss das Sensorausgangssignal auf Grund der nichtlinearen Mischregel auf eine lineare Mischregel umgerechnet werden, wobei das Sensorausgangssignal beispielsweise von einem analogen 4 - 20 mA Stromausgang geliefert wird, wie er bei vielen Sensoren vorhanden ist. Die Umrechnung auf eine lineare Mischregel kann z.B. anhand eines Polynoms erfolgen, hier konkret y = -3.00E-13·x$^6$ + 2.40E-10·x$^5$ - 8.43E-08·x$^4$ + 1.78E-05·x$^3$ - 2.66E-03·x$^2$ + 3.24E-01 x - 2.86, wobei y das Stromausgangssignal $I_S$ und x die Wärmeleitfähigkeit bedeuten. $PP_{min}$ = 4 mA entsprechen dann reinem $N_2$, $PP_{max}$ = 20 mA reinem $H_2$. Einsetzen des gemessenen Mischwertes von 76.42 mW m$^{-1}$ K$^{-1}$ für eine 50%/50%-Mischung ergibt einen Strom von 12.0 mA.

Diese drei Werte für den Strom können ins Gleichungssystem (6) eingesetzt werden. Für die Steigung der Kalibriergeraden CL in Fig. 4 folgt s = (100% - 0%)/(20 mA - 4 mA) = 6.25 %/mA). Eingesetzt in Gleichung (8),

$C_{1eff}$ · 6.25%/mA · (4 mA - 4 mA) + $C_{2eff}$ · 6.25%/mA · (20 mA - 4 mA) = 6.25%/mA - (12 mA - 4 mA)

$$C_{1eff} + C_{2eff} = 1$$

und weiter in Gleichung (9)

$$C_{1eff} \cdot 0\% + C_{2eff} \cdot 100\% = 50\%$$

$$C_{1eff} + C_{2eff} = 1$$

führt dies zu effektiven Konzentrationen von jeweils 50 mol% für $N_2$ und $H_2$.

Wenn im zweiten Ausführungsbeispiel das Erdgas aus zwei verschiedenen Quellen stammt, wobei das Erdgas aus der ersten Quelle eine Wärmeleitfähigkeit von 31.66 mW m$^{-1}$ K$^{-1}$ und das aus der zweiten Quelle eine Wärmeleitfähigkeit von 33.69 mW m$^{-1}$K$^{-1}$ hat, wird der Sensor linearisiert 5.22 mA bzw. 5.62 mA messen, was einer Wasserstoffkonzentration von (5.22 mA-4 mA)/(20 mA-4 mA) = 7.63% bzw. 10.13% $H_2$ in $N_2$ entspricht. Die beiden Erdgase enthalten von Natur aus jedoch keinen Wasserstoff. Nun muss zusätzlich der Messwert im Mischgas auf eine lineare Mischregel umgerechnet werden: dieser beträgt 82.19 mW m$^{-1}$ K$^{-1}$ für Erdgas aus der ersten Quelle bzw. 85.29 mW m$^{-1}$ K$^{-1}$ für Erdgas aus der zweiten Quelle, wiederum bei einer Zumischung von 50 mol% $H_2$, was ein Sensorsignal von 12.64 mA bzw. 12.97 mA ergibt. Oder als $H_2$-Konzentration ausgedrückt: 54.07% bzw. 56.14%. Dies sind nun die Werte, die ins Gleichungssystem (9) einzusetzen sind. Die Lösung, Gleichung (10), lautet für das Erdgas aus der ersten Quelle $C_{H2eff}$ = (54.00%-7.63%)/(100%-7.63%) = 50.20% und $C_{H2eff}$ = (56.06%-10.13%)/(100%-10.13%) = 51.11% für das Erdgas aus der zweiten Quelle. Ohne Umrechnung der Wärmeleitfähigkeitswerten auf eine lineare Mischregel, hätte sich für das Erdgas aus der ersten Quelle eine $H_2$-Konzentration von 32.8% bzw. 33.9% für das Erdgas aus der zweiten Quelle ergeben, also massiv falsch gegenüber der wirklichen Zumischung von 50 mol% $H_2$. Alle Gaseigenschaften sind im Beispiel für 25°C und 1013.25 mbar angegeben.

In einem dritten Ausführungsbeispiel wird das Konditionieren von Biomethan näher beschrieben. Dabei wird dem Biomethan Propan und/oder Luft zugemischt, um den Brennwert und/oder den Wobbeindex im nachgelagerten Erdgasnetz auf einen ortsüblichen Wert zu konditionieren. Da die Zusammensetzung des Biomethans sich ändern kann, z.B. weil diese von der Biomasse abhängig ist, die dem Fermenter der Biogasanlage zugeführt wird, ist es vorteilhaft, sowohl das Biomethan vor als auch das Mischgas nach der Zumischung von Propan/Luft zu messen, um die Zumischmenge von letzterem bestimmen zu können. Da die Eigenschaften von reinem Propan bzw. reiner Luft bekannt sind, braucht es für diese Gase keinen zusätzlichen Sensor: Das Beispiel steht also für den Fall n = 3, m =1. Es seien im Weiteren die Dichte $\rho$ und die Wärmeleitfähigkeit $\lambda$ die physikalischen Eigenschaften, die mit jeweils zwei entsprechenden Sensoren gemessen werden, deren Messbereiche Dichten von $\rho$ = 0.657 kg/m$^3$ (Methan, $CH_4$) bis $\rho$ =1.832 kg/m$^3$ (Propan) und Wärmeleitfähigkeiten von $\lambda$ = 18.31 mW m$^{-1}$ K$^{-1}$ (Propan) bis $\lambda$ = 33.96 mW m$^{-1}$ K$^{-1}$ (Methan, $CH_4$) umfassen.

Während für die Dichte von einer linearen Mischregel ausgegangen wird, d.h. die zu mischenden Gase als ideale Gase betrachtet werden (Abweichung zu realen Gasen machen im Beispiel nur 0.2% in der Dichte aus), unterliegt die Wärmeleitfähigkeit einer nichtlinearen Mischregel, weshalb auch in diesem Beispiel das Sensorausgangssignal auf einen lineare Mischregel umgerechnet werden muss, wobei das Sensorausgangssignal beispielsweise von einem analogen 4 - 20 mA Stromausgang geliefert wird, wie er bei vielen Sensoren vorhanden ist. Da der Wärmeleitfähigkeitsbereich etwas enger gefasst ist als im Beispiel der Wasserstoffzumischung oben, genügt hier ein Polynom 3. Ordnung: y = 1.75E-03·x$^3$ - 1.78E-01·x$^2$ + 6.63·x - 68.46, wobei y das Stromsignal $I_S$ und x die Wärmeleitfähigkeit bedeuten. Im Beispiel sollen zwei Biomethanmischungen so konditioniert werden, dass ein Brennwert von 33.6 MJ/m$^3$ und ein Wobbeindex von 40.3 MJ/m$^3$ resultiert.

**Tabelle 1 Konditionierung von Biomethan 1**

| Abkürzungen: WLF = Wärmeleitfähigkeit | | | | |
|---|---|---|---|---|
| *Die Werte für Luft und Propan werden nicht gemessen, sondern als bekannt vorausgesetzt.* | | | | |
| Gas/ physikalische Eigenschaft | Zusammensetzung Biomethan 1 | *Luft* | *Propan* | konditioniertes Biomethan 1 |
| $CH_4$ | 92% | | | 79.02% |
| $H_2$ | 0% | | | 0% |
| $N_2$ | 4% | | | 3.44% |
| $CO_2$ | 4% | | | 3.44% |
| Zumischung: | | | | |
| Propan | 0% | | | 5.10% |
| Luft | 0% | | | 9.00% |
| Brennwert [MJ/m$^3$] Wobbeindex [ MJ/m$^3$] | 33.55 42.96 | *0* *0* | *92.20* *74.13* | 33.52 40.30 |
| Dichte [kg/m$^3$] | 0.722 | *1.184* | *1.832* | 0.819 |

(fortgesetzt)

| Gas/ physikalische Eigenschaft | Zusammensetzung Biomethan 1 | Luft | Propan | konditioniertes Biomethan 1 |
|---|---|---|---|---|
| Abkürzungen: WLF = Wärmeleitfähigkeit |
| *Die Werte für Luft und Propan werden nicht gemessen, sondern als bekannt vorausgesetzt.* |
| Signal [mA] | 4.90 | *11.18* | *20.00* | 6.22 |
| $s \cdot (PP\text{-} PP_{min})$ s=100%/($I_s$-4mA) | 6.25%/mA· (4.90 - 4)mA = 5.62% | *6.25%/mA· (11.18-4)mA = 44.87%* | *6.25%/mA· (20.00-4)mA = 100%* | 6.25%/mA· (6.22 - 4) mA = 13.87% |
| WLF [mWm$^{-1}$K$^{-1}$] Signal [mA] | 32.90 19.32 | 26.25 *14.58* | *18.31* *4.00* | 30.98 18.13 |
| $s \cdot (PP\text{-} PP_{min})$ s=100%/($I_s$-4mA) | 6.25%/mA· (19.32 - 4) mA = 95.75% | *6.25%/mA· (14.58-4)mA = 66.12%* | *6.25%/mA· (4.00-4)mA = 0%* | 6.25%/mA· (18.13 - 4)mA = 88.31% |

Das Gleichungssystem (12) lautet damit nun:

$$C_{1eff} \cdot 5.62\% + C_{2eff} \cdot 44.87\% + C_{3eff} \cdot 100\% = 13.87\%$$

$$C_{1eff} \cdot 95.75\% + C_{2eff} \cdot 66.12\% + C_{3eff} \cdot 0\% = 88.31\%$$

$$C_{1eff} + C_{2eff} + C_{3eff} = 100\%$$

Als Lösung dieses Gleichungssystems erhält man $C_{1eff}$ = 85.9% Biomethan 1, $C_{2eff}$ = 9.12% Luft und $C_{3eff}$ = 4.95% Propan, woraus sich ein Brennwert von 33.39 MJ/m$^3$ und ein Wobbeindex von 40.25 MJ/m$^3$ ergibt. Mit den (wahren) Werten in Tabelle 1 verglichen: 85.9% Biomethan 1, 9.00% Luft und 5.1% Propan.

**Tabelle 2 Konditionierung von Biomethan 2**

| Gas/ physikalische Eigenschaft | Zusammensetzung Biomethan 2 | Luft | Propan | konditioniertes Biomethan 2 |
|---|---|---|---|---|
| Abkürzungen: WLF = Wärmeleitfähigkeit |
| *Die Werte für Luft und Propan werden nicht gemessen, sondern als bekannt vorausgesetzt.* |
| CH$_4$ | 94% | | | 72.48% |
| H$_2$ | 4% | | | 3.08% |
| N$_2$ | 0% | | | 0% |
| CO$_2$ | 2% | | | 1.54% |
| Zumischung: | | | | |
| Propan | 0% | | | 7.40% |
| Luft | 0% | | | 15.50% |
| Brennwert [MJ/m$^3$] Wobbeindex [ MJ/m$^3$] | 34.74 46.65 | *0* *0* | *92.20* *74.13* | 33.61 40.30 |
| Dichte [kg/m$^3$] Signal [mA] | 0.657 4.00 | *1.184* *11.18* | *1.832* *20.00* | 0.824 6.27 |
| $s \cdot (PP\text{-}PP_{min})$ s=100%/($I_s$-4mA) | 6.25%/mA· (4.00 - 4)mA = 0% | *6.25%/mA· (11.18-4)mA = 44.87%* | *6.25%/mA· (20.00 - 4)mA = 100%* | 6.25%/mA· (6.27 - 4)mA = 14.19% |

(fortgesetzt)

| Abkürzungen: WLF = Wärmeleitfähigkeit | | | | |
|---|---|---|---|---|
| *Die Werte für Luft und Propan werden nicht gemessen, sondern als bekannt vorausgesetzt.* | | | | |
| Gas/ physikalische Eigenschaft | Zusammensetzung Biomethan 2 | *Luft* | *Propan* | konditioniertes Biomethan 2 |
| WLF [mWm$^{-1}$ K$^{-1}$] <br> Signal [mA] | 36.68 <br> 21.60 | *26.25* <br> *14.58* | *18.31* <br> *4.00* | 32.77 <br> 19.24 |
| $s \cdot (PP\text{-} PP_{min})$ <br> s=100%/($I_s$-4mA) | 6.25%/mA· (21.60 - 4) mA = 110.0% | *6.25%/mA· (14.58-4)mA = 66.12%* | *6.25%/mA· (4.00 - 4)mA = 0%* | 6.25%/mA. (19.24 - 4)mA = 95.25% |

Das Gleichungssystem (12) lautet damit nun:

$$C_{1eff} \cdot 0\% + C_{2eff} \cdot 44.87\% + C_{3eff} \cdot 100\% = 14.19\%$$

$$C_{1eff} \cdot 110.0\% + C_{2eff} \cdot 66.12\% + C_{3eff} \cdot 0\% = 95.25\%$$

$$C_{1eff} + C_{2eff} + C_{3eff} = 100\%$$

Als Lösung dieses Gleichungssystems erhält man $C_{1eff}$ = 77.16% Biomethan 2, $C_{2eff}$ = 15.68% Luft und $C_{3eff}$ = 7.16% Propan, woraus sich ein Brennwert von 33.40 MJ/m$^3$ und ein Wobbeindex von 40.22 MJ/m$^3$ ergibt. Mit den (wahren) Werten in Tabelle 2 verglichen: 77.1% Biomethan 2, 15.5% Luft und 7.4% Propan. Biomethan 2 hat eine Wärmeleitfähigkeit, die ausserhalb des 4 - 20 mA Bereichs liegt, was zu Konzentrationswerten von über 100% führt. In der Praxis müsste ein Wärmeleitfähigkeitssensor gewählt werden, der eine grössere Messspanne hat als hier im Beispiel. Es ist aber interessant zu sehen, dass das zugrunde liegende Berechnungsprinzip auch mit nicht physikalischen Werten die richtigen effektiven Konzentrationen liefert. Alle Gaseigenschaften sind im Beispiel für 25 °C und 101 3.25 mbar gegeben.
Fig. 8 zeigt eine Ausführungsvariante für das gleichzeitige Mischen von n Gasen G1, ..., Gn, die einer Gasmischeinheit 4 zugeführt werden. Im Verfahren gemäss vorliegender Erfindung werden im gemischten Gas Gmix$_{1...n}$ mit n - 1 Sensoren 3.1,..., *n-1* n - 1 physikalische Eigenschaften $PP_{mix,1}$, ..., $PP_{mix.n-1}$ gemessen. In der gezeigten Ausführungsvariante wird davon ausgegangen, dass die Zusammensetzung der zu mischenden Gase G1, ..., Gn Schwankungen unterliegen, weshalb die n - 1 physikalischen Eigenschaften $PP_{1.1}$, ... , $PP_{n.n-1}$ mit n·(n-1) Sensoren 3.1.*1,..., n - 1,* ..., 3.n.1,..., *n-1* jeweils auch in den zu mischenden Gasen gemessen werden.
Wenn wie im Beispiel der in Fig. 9 gezeigten Ausführungsvariante sequentiell gemischt wird, kann auch mit nur einer physikalischen Gaseigenschaft das Mischen von n Eingangsgasströmen überwacht werden: Gas G1 wird zuerst mit Gas G2 gemischt, diese Mischung dann mit Gas G3 und so fort, bis am Schluss alle n Gase gemischt sind. Dazu werden n - 1 Gasmischeinheiten 4.1, ..., 4.n-1 und maximal 2n -1 Sensoren benötigt, denn jeder Sensor 3.12 ... 3.1-n nach einer der ersten n -2 Mischstufen und Gasmischeinheiten 4.1 ...4.n-2 in Fig. 9 dient gleichzeitig als Sensor für eines der Gase in der nächsten Mischstufe. Ist das Zumischgas in einer der Mischstufen von vornherein bekannt, braucht es keinen zusätzlichen Sensor (vgl. Fig. 2). Die Minimalanzahl von Sensoren ist im Fall, dass alle Zumischgase bekannt sind, gleich n-1.
Fig. 10 zeigt eine Ausführungsvariante des kaskadierten Mischens: Die n Gase werden in einer ersten Stufe paarweise gemischt, diese Mischungen in einer zweiten Stufe wiederum paarweise, bis am Schluss alle Gase gemischt sind. Wiederum werden maximal 2n - 1 und minimal n - 1 Sensoren benötigt. Dazu kommen wieder n - 1 Gasmischeinheiten.
Weitere Ausführungsvarianten ergeben sich aus der Kombination von sequentiellem und kaskadiertem Mischen und/oder indem zum Beispiel beim sequentiellen Mischen in jeder Stufe drei Gase gemischt werden, wobei in diesem Fall jeweils Sensoren für zwei physikalische Eigenschaften vorgesehen werden müssen.
Fig. 11 zeigt ein Beispiel einer Mischvorrichtung für das Mischen zweier Gase 1.1 und 1.2 zusammen mit einem Ausführungsbeispiel einer Messvorrichtung zur Bestimmung der Mischanteile gemäss vorliegender Erfindung. Die Mischvorrichtung 9a oder 9b enthält eine Gasmischeinheit 4, zwei Gaszuführungen 5.1 und 5.2 zum Zuführen der zu mischenden Gase 1.1 und 1.2 und eine Gasausgangsleitung 6 für das gemischte Gas 2.
Die Messvorrichtung des Ausführungsbeispiels umfasst jeweils einen Sensor 3.1, 3.2 zum Messen einer physikalischen Eigenschaft der zwei zugeführten Gase 1.1 und 1.2, einen dritten Sensor 3 zum Messen derselben physikalischen Eigenschaft im gemischten Gas und eine Auswerteeinheit 8, die mit den drei Sensoren verbunden ist, und

**EP 4 169 607 B1**

die zur Ausführung eines Verfahrens gemäss vorliegender Erfindung oder einer der oben beschriebenen Ausführungsformen oder -varianten eingerichtet ist.

Die Sensoren 3.1 und 3.2 für die zu mischenden Gase können beispielsweise in den Gaszuführungen 5.1 und 5.2 angeordnet sein und der dritte Sensor 3 für das gemischte Gas in der Gasausgangsleitung 6. Die Auswerteeinheit 8 kann wahlweise separat oder in einer Rechen- oder Steuereinheit der Mischvorrichtung 9a ausgebildet sein.

Wenn die physikalische Eigenschaft ohne Einbusse der Genauigkeit über einem grossen Wertebereich kalibriert werden kann, können die Sensoren universell eingesetzt werden: Die Kalibrierkurve für den scheinbaren Mischanteil deckt dann einen sehr grossen Bereich möglicher Gaszusammensetzungen ab. Damit lassen sich Produktions- und Lagerhaltungskosten senken, da nur ein Sensortyp pro physikalische Eigenschaft gebaut und gelagert werden muss. So sind beispielsweise Sensoren, die für die Qualitätssicherung von Biogas gedacht sind (Gemisch aus Kohlenstoffdioxid, $CO_2$ und Methan, $CH_4$), auch für das Mischen von Nitrox Atemgasgemische für Taucher (Gemisch aus Stickstoff, $N_2$ und Sauerstoff, $O_2$) einsetzbar, wenn die physikalische Eigenschaft entweder die Dichte oder die Wärmeleitfähigkeit ist.

**Patentansprüche**

1. Verfahren zur Bestimmung der Mischanteile beim Mischen von n = 2, 3, 4 oder mehr Gasen, in welchem im gemischten Gas und in einem oder mehreren der n Gase jeweils mindestens n - 1 physikalische Eigenschaften gemessen werden, wobei

   • die n - 1 physikalischen Eigenschaften mit mindestens zwei Sensoren gemessen werden, und
   • aus den Werten der gemessenen physikalischen Eigenschaft oder Eigenschaften die Mischanteile der n Gase im gemischten Gas bestimmt werden,
   **dadurch gekennzeichnet, dass** die n - 1 physikalischen Eigenschaften im gemischten Gas mit Sensoren gemessen werden, auf welchen jeweils für die betreffende physikalische Eigenschaft eine Kalibrierkurve für die Bestimmung eines scheinbaren Mischanteils hinterlegt ist, d.h. eines Mischanteils für eine Gaszusammensetzung, die nicht der effektiv vorhandenen Gaszusammensetzung zu entsprechen braucht, und
   die n - 1 physikalischen Eigenschaften in einem oder mehreren der n Gase mit Sensoren gemessen werden, auf welchen jeweils für die betreffende physikalische Eigenschaft eine Kalibrierkurve für die Bestimmung eines scheinbaren Mischanteils hinterlegt ist, wobei diese identisch ist mit der Kalibrierkurve in dem für die Messung der betreffenden physikalischen Eigenschaft im gemischten Gas verwendeten Sensor,
   und aus den derart bestimmten scheinbaren Mischanteilen für jedes der n Gase der effektive Mischanteil im gemischten Gas bestimmt wird.

2. Verfahren nach Anspruch 1, in welchem mindestens eine physikalische Eigenschaft sowohl im gemischten Gas als auch in mindestens einem der n Gase oder mindestens zwei physikalische Eigenschaften im gemischten Gas gemessen werden.

3. Verfahren nach Anspruch 1 oder 2, in welchem für diejenigen physikalischen Eigenschaften, für die eine lineare Beziehung zwischen der im gemischten Gas gemessenen physikalischen Eigenschaft einerseits und der entsprechenden physikalischen Eigenschaft und den Mischanteilen der n Gase andererseits besteht oder für die die Abweichung von der Linearität im Bereich, in dem gemessen wird, innerhalb eines Toleranzbereichs von 0.2% oder 0.5% oder 1% liegt, die entsprechende lineare Beziehung für die Bestimmung der Mischanteile verwendet wird.

4. Verfahren nach einem der Ansprüche 1 bis 3, in welchem für diejenigen physikalischen Eigenschaften, für die keine lineare Beziehung zwischen der im gemischten Gas gemessenen physikalischen Eigenschaft einerseits und der entsprechenden physikalischen Eigenschaft und den Mischanteilen der n Gase andererseits besteht und für die die Abweichung von der Linearität im Bereich, in dem gemessen wird, ausserhalb eines Toleranzbereichs von 0.2% oder 0.5% oder 1% liegt, die Werte der betreffenden physikalischen Eigenschaft durch Funktionswerte ersetzt werden, die durch Einsetzen ersterer in eine nichtlineare Funktion bestimmt werden, beispielsweise durch Einsetzen in ein Polynom zweiten, dritten oder höheren Grades, so dass der Funktionswert der im gemischten Gas gemessenen physikalischen Eigenschaft linear von den Funktionswerten der betreffenden physikalischen Eigenschaft und den Mischanteilen der n Gase abhängt, und für die Bestimmung der Mischanteile jeweils die lineare Beziehung zwischen dem Funktionswert der im gemischten Gas gemessenen physikalischen Eigenschaft und den Funktionswerten der entsprechenden physikalischen Eigenschaft und den Mischanteilen der n Gase verwendet wird.

5. Verfahren nach einem der Ansprüche 1 bis 4, in welchem für diejenigen physikalischen Eigenschaften, für die eine lineare Beziehung zwischen der im gemischten Gas gemessenen physikalischen Eigenschaft einerseits und der entsprechenden physikalischen Eigenschaft und den Mischanteilen der n Gase andererseits verwendet wird, die im gemischten Gas gemessene physikalische Eigenschaft als Linearkombination der entsprechenden physikalischen Eigenschaft der n Gase betrachtet wird und für diejenigen physikalischen Eigenschaften, für die keine lineare Beziehung zwischen der im gemischten Gas gemessenen physikalischen Eigenschaft einerseits und der entsprechenden physikalischen Eigenschaft und den Mischanteilen der n Gase andererseits verwendet wird, der Funktionswert, der aus dem Wert der im gemischten Gas gemessenen physikalischen Eigenschaft bestimmt wird, als Linearkombination der aus den Werten der entsprechenden physikalischen Eigenschaft der n Gase bestimmten Funktionswerte betrachtet wird, wobei für alle n - 1 physikalischen Eigenschaften die Mischanteile den Koeffizienten der Linearkombinationen entsprechen, und aus den n - 1 Linearkombinationen und aus der Kenntnis, dass die Summe der Mischanteile gleich eins oder 100 % ist, die Mischanteile bestimmt werden.

6. Verfahren nach einem der Ansprüche 1 bis 5, in welchem für diejenigen der n Gase, in denen keine oder nicht alle der n - 1 physikalischen Eigenschaften gemessen werden, bei der Bestimmung der Mischanteile für die nicht gemessenen physikalischen Eigenschaften oder für die aus denselben bestimmten Funktionswerte vorgegebene Werte eingesetzt werden, wobei die vorgegebenen Werte beispielsweise aus der Zusammensetzung der betreffenden Gase bestimmt werden.

7. Verfahren gemäss einem der Ansprüche 1 bis 6, wobei als physikalische Eigenschaft oder Eigenschaften die Wärmeleitfähigkeit, die Wärmekapazität, die Dichte, die Viskosität, die Schallgeschwindigkeit, der Diffusionskoeffizient, der Brechungsindex, die Dielektrizitätskonstante und/oder der optische Absorptionskoeffizient bei einer bestimmten Wellenlänge elektromagnetischer Strahlung gemessen werden.

8. Verfahren nach einem der Ansprüche 1 bis 7, in welchem für diejenigen der n Gase, in denen keine oder nicht alle der n - 1 physikalischen Eigenschaften gemessen werden, für die scheinbaren Mischanteile vorgegebene Werte eingesetzt werden, wobei die vorgegebenen Werte beispielsweise aus der Zusammensetzung der betreffenden Gase bestimmt werden.

9. Verfahren nach einem der Ansprüche 1 bis 8, wobei Werte von physikalischen Eigenschaften, die nichtlinearen Mischregeln unterliegen, zuerst mit Hilfe einer nichtlinearen Funktion umgerechnet werden, so dass die Funktionswerte oder entsprechenden Sensorsignale linearen Mischregeln unterliegen.

10. Verfahren nach einem der Ansprüche 1 bis 9, wobei $n \geq 3$ Gase sequentiell gemischt werden, indem zuerst zwei Gase gemischt werden und danach das Mischgas in einem weiteren Zweigasmischprozess mit einem weiteren Gas gemischt wird und das Mischen des zuletzt gemischten Mischgases mit einem weiteren Gas wiederholt wird, bis alle n Gase miteinander gemischt sind, und wobei alle Sensoren identisch für dieselbe physikalische Eigenschaft kalibriert sind.

11. Verfahren nach einem der Ansprüche 1 bis 9, wobei $n \geq 4$ Gase jeweils zu zweien in einer ersten und danach die jeweiligen Mischgase in einer oder mehreren weiteren Kaskaden zu zweien gemischt werden, bis alle n Gase gemischt sind, und wobei alle Sensoren identisch für dieselbe physikalische Eigenschaft kalibriert sind.

12. Verfahren nach einem der Ansprüche 1 bis 9, wobei das Mischen der $n \geq 4$ Gase in einer Kombination von sequentiellem und kaskadiertem Mischen erfolgt, und alle Sensoren identisch für dieselbe physikalische Eigenschaft kalibriert sind.

13. Messvorrichtung zur Bestimmung der Mischanteile beim Mischen von n = 2, 3, 4 oder mehr Gasen, umfassend Sensoren zum Messen von n - 1 physikalischen Eigenschaften im gemischten und in einem oder mehreren der n Gase und eine Auswerteeinheit, die mit den Sensoren verbunden ist, und die zur Ausführung eines Verfahrens gemäss einem der Ansprüche 1 bis 12 eingerichtet ist.

14. Messvorrichtung nach Anspruch 13, wobei die Auswerteeinheit in einer Rechen- oder Steuereinheit einer Mischvorrichtung ausgebildet ist, oder wobei die Messvorrichtung und/oder einer oder mehrere der Sensoren als mobil einsetzbare Geräte ausgebildet sind.

**Claims**

1. Method for determining the mixing proportions when mixing n = 2, 3, 4 or more gases, in which at least n - 1 physical properties are measured in each case in the mixed gas and in one or more of the n gases, wherein

> • the n - 1 physical properties are measured using at least two sensors, and
> • the mixing proportions of the n gases in the mixed gas are determined from the values of the measured physical property or properties,

**characterized in that** the n - 1 physical properties in the mixed gas are measured using sensors on which a calibration curve for determining an apparent mixing proportion, i.e. a mixing proportion for a gas composition which does not need to correspond to the effectively present gas composition, is stored in each case for the relevant physical property, and the n - 1 physical properties in one or more of the n gases are measured using sensors on which a calibration curve for determining an apparent mixing proportion is stored in each case for the relevant physical property, wherein said calibration curve is identical to the calibration curve in the sensor used for measuring the relevant physical property in the mixed gas, and the effective mixing proportion in the mixed gas is determined from the apparent mixing proportions determined in this way for each of the n gases.

2. Method according to claim 1, in which at least one physical property is measured both in the mixed gas and in at least one of the n gases or at least two physical properties are measured in the mixed gas.

3. Method according to claim 1 or 2, in which, for those physical properties for which there is a linear relationship between the physical property measured in the mixed gas, on the one hand, and the corresponding physical property and the mixing proportions of the n gases, on the other hand, or for which the deviation from the linearity in the range in which measurement is carried out lies within a tolerance range of 0.2% or 0.5% or 1%, the corresponding linear relationship is used for determining the mixing proportions.

4. Method according to any one of claims 1 to 3, in which, for those physical properties for which there is no linear relationship between the physical property measured in the mixed gas, on the one hand, and the corresponding physical property and the mixing proportions of the n gases, on the other hand, and for which the deviation from the linearity in the range in which measurement is carried out lies outside a tolerance range of 0.2% or 0.5% or 1%, the values of the relevant physical property are replaced by functional values which are determined by inserting the former into a nonlinear function, for example by inserting into a polynomial of the second, third or higher degree, such that the functional value of the physical property measured in the mixed gas depends linearly on the functional values of the relevant physical property and the mixing proportions of the n gases, and the linear relationship between the functional value of the physical property measured in the mixed gas and the functional values of the corresponding physical property and the mixing proportions of the n gases is used in each case for determining the mixing proportions.

5. Method according to any one of claims 1 to 4, in which, for those physical properties for which a linear relationship between the physical property measured in the mixed gas, on the one hand, and the corresponding physical property and the mixing proportions of the n gases, on the other hand, is used, the physical property measured in the mixed gas is considered as a linear combination of the corresponding physical property of the n gases, and, for those physical properties for which no linear relationship between the physical property measured in the mixed gas, on the one hand, and the corresponding physical property and the mixing proportions of the n gases, on the other hand, is used, the functional value which is determined from the value of the physical property measured in the mixed gas is considered as a linear combination of the functional values determined from the values of the corresponding physical property of the n gases, wherein, for all n - 1 physical properties, the mixing proportions correspond to the coefficients of the linear combinations, and the mixing proportions are determined from the n - 1 linear combinations and from the knowledge that the sum of the mixing proportions is equal to one or 100%.

6. Method according to any one of claims 1 to 5, in which, for those of the n gases in which no or not all of the n - 1 physical properties are measured, predetermined values are used in the determination of the mixing proportions for the non-measured physical properties or for the functional values determined from the same, wherein the predetermined values are determined, for example, from the composition of the relevant gases.

7. Method according to any one of claims 1 to 6, wherein the physical property or properties measured are the thermal conductivity, the heat capacity, the density, the viscosity, the speed of sound, the diffusion coefficient, the refractive index, the dielectric constant and/or the optical absorption coefficient at a particular wavelength of electromagnetic radiation.

8. Method according to any one of claims 1 to 7, in which, for those of the n gases in which no or not all of the n - 1 physical properties are measured, predetermined values are used for the apparent mixing proportions, wherein the predetermined values are determined, for example, from the composition of the relevant gases.

9. Method according to any one of claims 1 to 8, wherein values of physical properties which are subject to nonlinear mixing rules are first converted with the aid of a nonlinear function, such that the functional values or corresponding sensor signals are subject to linear mixing rules.

10. Method according to any one of claims 1 to 9, wherein $n \geq 3$ gases are mixed sequentially by first mixing two gases and then mixing the mixed gas with a further gas in a further two-gas mixing process and repeating the mixing of the last-mixed mixed gas with a further gas until all n gases are mixed with one another, and wherein all sensors are calibrated identically for the same physical property.

11. Method according to any one of claims 1 to 9, wherein $n \geq 4$ gases are respectively mixed into two in a first cascade and then the respective mixed gases are mixed into two in one or more further cascades until all n gases are mixed, and wherein all sensors are calibrated identically for the same physical property.

12. Method according to any one of claims 1 to 9, wherein the mixing of the $n \geq 4$ gases is effected in a combination of sequential and cascaded mixing, and all sensors are calibrated identically for the same physical property.

13. Measuring device for determining the mixing proportions when mixing n = 2, 3, 4 or more gases, comprising sensors for measuring n - 1 physical properties in the mixed gas and in one or more of the n gases and an evaluation unit which is connected to the sensors and which is set up to carry out a method according to any one of claims 1 to 12.

14. Measuring device according to claim 13, wherein the evaluation unit is realized in a computing unit or control unit of a mixing device, or wherein the measuring device and/or one or more of the sensors are designed as mobile appliances.

**Revendications**

1. Procédé de détermination des proportions de mélange lors du mélange de n = 2, 3, 4 gaz ou plus, dans lequel, dans le gaz mélangé et dans un ou plusieurs des n gaz, on mesure respectivement au moins n - 1 propriétés physiques, dans lequel

   • on mesure les n - 1 propriétés physiques avec au moins deux capteurs, et
   • à partir des valeurs de la propriété physique ou des propriétés physiques mesurées, on détermine les proportions de mélange des n gaz dans le gaz mélangé,
   **caractérisé en ce que** l'on mesure les n - 1 propriétés physiques dans le gaz mélangé avec des capteurs sur lesquels, respectivement pour la propriété physique concernée, est déposée une courbe d'étalonnage pour la détermination d'une proportion de mélange apparente, c'est-à-dire d'une proportion de mélange pour une composition de gaz qui ne doit pas correspondre à la composition de gaz effectivement présente, et
   l'on mesure les n - 1 propriétés physiques dans un ou plusieurs des n gaz avec des capteurs sur lesquels, respectivement pour la propriété physique concernée, est déposée une courbe d'étalonnage pour la détermination d'une proportion de mélange apparente, celle-ci étant identique à la courbe d'étalonnage dans le capteur utilisé pour la mesure de la propriété physique concernée dans le gaz mélangé,
   et à partir des proportions de mélange apparentes ainsi déterminées, on détermine pour chacun des n gaz la proportion de mélange effective dans le gaz mélangé.

2. Procédé selon la revendication 1, dans lequel on mesure au moins une propriété physique aussi bien dans le gaz mélangé que dans au moins un des n gaz ou au moins deux propriétés physiques dans le gaz mélangé.

3. Procédé selon la revendication 1 ou 2, dans lequel, pour les propriétés physiques pour lesquelles il existe une relation linéaire entre la propriété physique mesurée dans le gaz mélangé d'une part et la propriété physique correspondante et les proportions de mélange des n gaz d'autre part ou pour lesquelles l'écart par rapport à la linéarité dans la plage dans laquelle on mesure se situe dans une plage de tolérance de 0,2 % ou 0,5 % ou 1 %, on utilise la relation linéaire correspondante pour la détermination des proportions de mélange.

4. Procédé selon l'une quelconque des revendications 1 à 3, dans lequel, pour les propriétés physiques pour lesquelles il n'existe pas de relation linéaire entre la propriété physique mesurée dans le gaz mélangé d'une part et la propriété physique correspondante et les proportions de mélange des n gaz d'autre part et pour lesquelles l'écart par rapport à la linéarité dans la plage dans laquelle on mesure se situe en dehors d'une plage de tolérance de 0,2 % ou 0,5 % ou 1 %, on remplace les valeurs de la propriété physique concernée par des valeurs de fonction qui sont déterminées par insertion de la première dans une fonction non linéaire, par exemple par insertion dans un polynôme de deuxième, troisième degré ou plus, de sorte que la valeur de fonction de la propriété physique mesurée dans le gaz mélangé dépend linéairement des valeurs de fonction de la propriété physique concernée et des proportions de mélange des n gaz, et pour la détermination des proportions de mélange, on utilise respectivement la relation linéaire entre la valeur de fonction de la propriété physique mesurée dans le gaz mélangé et les valeurs de fonction de la propriété physique correspondante et des proportions de mélange des n gaz.

5. Procédé selon l'une quelconque des revendications 1 à 4, dans lequel, pour les propriétés physiques pour lesquelles on utilise une relation linéaire entre la propriété physique mesurée dans le gaz mélangé d'une part et la propriété physique correspondante et les proportions de mélange des n gaz d'autre part, on considère la propriété physique mesurée dans le gaz mélangé comme une combinaison linéaire de la propriété physique correspondante des n gaz, et pour les propriétés physiques pour lesquelles on n'utilise pas de relation linéaire entre la propriété physique mesurée dans le gaz mélangé d'une part et la propriété physique correspondante et les proportions de mélange des n gaz d'autre part, on considère la valeur de fonction qui est déterminée à partir de la valeur de la propriété physique mesurée dans le gaz mélangé comme une combinaison linéaire des valeurs de fonction déterminées à partir des valeurs de la propriété physique correspondante des n gaz, dans lequel, pour toutes les n - 1 propriétés physiques, les proportions de mélange correspondent aux coefficients des combinaisons linéaires, et à partir des n - 1 combinaisons linéaires et de la connaissance que la somme des proportions de mélange est égale à un ou 100 %, on détermine les proportions de mélange.

6. Procédé selon l'une quelconque des revendications 1 à 5, dans lequel, pour ceux des n gaz dans lesquels on ne mesure pas ou pas toutes les n - 1 propriétés physiques, on utilise, lors de la détermination des proportions de mélange, pour les propriétés physiques non mesurées ou pour les valeurs prédéfinies à partir des mêmes valeurs de fonction déterminées, dans lequel les valeurs prédéfinies sont déterminées par exemple à partir de la composition des gaz concernés.

7. Procédé selon l'une quelconque des revendications 1 à 6, dans lequel on mesure comme propriété physique ou propriétés physiques la conductivité thermique, la capacité thermique, la densité, la viscosité, la vitesse du son, le coefficient de diffusion, l'indice de réfraction, la constante diélectrique et/ou le coefficient d'absorption optique à une longueur d'onde déterminée de rayonnement électromagnétique.

8. Procédé selon l'une quelconque des revendications 1 à 7, dans lequel, pour ceux des n gaz dans lesquels on ne mesure pas ou pas toutes les n - 1 propriétés physiques, on utilise pour les proportions de mélange apparentes des valeurs prédéfinies, dans lequel les valeurs prédéfinies sont déterminées par exemple à partir de la composition des gaz concernés.

9. Procédé selon l'une quelconque des revendications 1 à 8, dans lequel des valeurs de propriétés physiques qui sont soumises à des règles de mélange non linéaires sont d'abord converties à l'aide d'une fonction non linéaire, de sorte que les valeurs de fonction ou des signaux de capteur correspondants sont soumis à des règles de mélange linéaires.

10. Procédé selon l'une quelconque des revendications 1 à 9, dans lequel $n \geq 3$ gaz sont mélangés séquentiellement en mélangeant d'abord deux gaz et en mélangeant ensuite le gaz mélangé avec un autre gaz dans un autre processus de mélange à deux gaz et en répétant le mélange du gaz mélangé mélangé en dernier avec un autre gaz jusqu'à ce que tous les n gaz soient mélangés les uns avec les autres, et dans lequel tous les capteurs sont étalonnés de manière identique pour la même propriété physique.

11. Procédé selon l'une quelconque des revendications 1 à 9, dans lequel $n \geq 4$ gaz sont mélangés respectivement en deux dans une première et ensuite les gaz mélangés respectifs sont mélangés en deux dans une ou plusieurs autres cascades jusqu'à ce que tous les n gaz soient mélangés, et dans lequel tous les capteurs sont étalonnés de manière identique pour la même propriété physique.

12. Procédé selon l'une quelconque des revendications 1 à 9, dans lequel le mélange des $n \geq 4$ gaz a lieu dans une combinaison de mélange séquentiel et en cascade, et tous les capteurs sont étalonnés de manière identique pour la même propriété physique.

**13.** Dispositif de mesure pour déterminer les proportions de mélange lors du mélange de n = 2, 3, 4 gaz ou plus, comprenant des capteurs pour mesurer n - 1 propriétés physiques dans le gaz mélangé et dans un ou plusieurs des n gaz et une unité d'évaluation qui est reliée aux capteurs et qui est conçue pour mettre en oeuvre un procédé selon l'une quelconque des revendications 1 à 12.

**14.** Dispositif de mesure selon la revendication 13, dans lequel l'unité d'évaluation est réalisée dans une unité de calcul ou de commande d'un dispositif de mélange, ou dans lequel le dispositif de mesure et/ou un ou plusieurs des capteurs sont réalisés sous forme d'appareils pouvant être utilisés de manière mobile.

Fig. 1

Fig. 2

Fig. 3

Fig. 4

EP 4 169 607 B1

Fig. 5

EP 4 169 607 B1

Fig. 6

$$y = -0.0625x - 0.25$$

$$y = -0.01x^2 + 1.4288x - 17.003$$

Fig. 7

Fig. 8

EP 4 169 607 B1

Fig. 9

Fig. 10

EP 4 169 607 B1

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0240992 A1
- EP 0472131 A1

- EP 2667276 A
- WO 0240992 A